# EUROPEAN PATENT SPECIFICATION

(11) **EP 1 261 588 B1**
(45) Date of publication and mention of the grant of the patent: **20.10.2004**
(21) Application number: 01942360.7
(22) Date of filing: 12.01.2001
(51) Int. Cl.: C07D 233/56, C07D 233/64, C07D 405/06, A61K 31/4174, A61K 31/4178, A61P 3/06, A61P 3/10, A61P 9/02, A61P 15/00, A61P 25/00

(54) **IMIDAZOLE COMPOUNDS AS Alpha2-ADRENOCEPTORS ANTAGONISTS**
IMIDAZOLVERBINDUNGEN ALS Alpha2-ADRENOREZEPTOREN ANTAGONISTEN
COMPOSES D'IMIDAZOLE UTILISES COMME ANTAGONISTES DES RECEPTEURS alpha2- ADRENERIQUES

(30) Priority: 14.01.2000 FI 20000073
(43) Date of publication of application: 04.12.2002
(73) Proprietor: ORION CORPORATION, 02200 Espoo (FI)
(72) Inventor: HUHTALA, Paavo, FIN-02200 Espoo (FI); KARJALAINEN, Arto, FIN-02600 Espoo (FI); HAAPALINNA, Antti, FIN-20360 Turku (FI); LEHTIMÄKI, Jyrki, FIN-21570 Sauvo (FI); KARJALAINEN, Arja, FIN-02210 Espoo (FI); VIRTANEN, Raimo, FIN-21290 Rusko (FI)
(74) Representative: Sexton, Jane Helen
(86) International application number: PCT/FI2001/000030
(87) International publication number: WO 2001/051472

(56) References cited:
- WO-A-00/07997
- WO-A-01/00586
- WO-A-97/12874
- WO-A-99/28300
- GB-A- 2 225 782
- US-A- 5 434 177
- US-A- 5 498 623
- US-A- 5 541 211

## Description

### BACKGROUND OF THE INVENTION

The present invention relates to new pharmacologically active imidazole derivatives and pharmaceutically acceptable salts and esters thereof, as well as to processes for their preparation and to pharmaceutical compositions containing them.

It is known that several derivatives of imidazole have affinity for alpha1 and/or alpha2 adrenoceptors. Accordingly, i.a. WO-A-97 12874 describes imidazole-substituted (1,2,3,4-tetrahydro-1-naphthalenyl)- and (2,3-dihydro-1H-inden-1-yl)-derivatives which are stated to possess affinity for alpha2 adrenoceptors most of them being selective alpha2 adrenoceptor agonists. EP-A-0 717 037 describes 4-(1,2,3,4-tetrahydro-1-naphthalenyl)- and 4-(2,3-dihydro-1H-inden-1-yl)-1H-imidazole derivatives which possess alfa2 adrenoceptor agonistic and alpha1 adrenoceptor antagonistic activity. On the other hand the imidazole derivatives disclosed in EP-A-0 183 492 are known as selective alpha2 adrenoceptor antagonists. Compounds acting on the said alpha adrenoceptors may exert a wide variety of peripheral and/or CNS (central nervous system) effects in mammals.

### SUMMARY OF THE INVENTION

The inventors have now found that the present imidazole derivatives of the invention exhibit affinity for alpha2 adrenoceptors so that they can be useful in the treatment of various disorders or diseases wherein the alpha2 adrenoceptors are involved. Such disorders or diseases include various disorders of the central nervous system (CNS), i.e. neurological, psychiatric or cognition disorders, as well as various disorders of the pheripheric system, e.g. diabetes, orthostatic hypotension, lipolytic disorders (such as obesity) or sexual dysfunction.

### DETAILED DESCRIPTION OF THE INVENTION

The imidazole derivatives of the invention can be represented by the following formula (I): 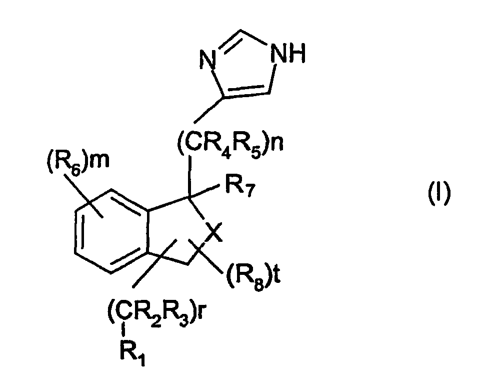 wherein
X is -CH₂-(CH₂)ₚ-, -O-, =NH or -S-;
R₁ is phenyl, naphthyl, 1,2,3,4-tetrahydronaphthyl, C₃-C₇-cycloalkyl, C₅ -C₇-cycloalkenyl, C₅ -C₇-cycloalkynyl or mono- or bicyclic aromatic or partially or fully saturated heterocyclic group with 5 to 10 ring atoms which consist of carbon atoms and one to three heteroatoms selected from N, O and S;
wherein the said phenyl, naphthyl,1,2,3,4-tetrahydronaphthyl, C₃ -C₇-cycloalkyl, C₅ -C₇-cycloalkenyl, C₅ -C₇-cycloalkynyl or mono- or bicyclic aromatic or partially or fully saturated heterocyclic group is optionally substituted with one to three substituents selected independently from halogen, -OH, -NH₂, halo-C₁-C₆-alkyl, C₁-C₆-alkyl, C₂-C₆-alkenyl, C₂-C₆-alkynyl, C₁-C₆-alkoxy, OH-(C₁-C₆)-alkyl, NH₂-(C₁-C₆)-alkyl and mono- or di(C₁-C₆-alkyl)amino;
R₂ is H or C₁-C₆-alkyl;
R₃ is H or C₁-C₆-alkyl; and
R₄ is H or C₁-C₆-alkyl;
R₅ is H, or R₅ and R₇ form together a bond;
each R₆ is independently halogen, -OH, -NH₂, halo-C₁-C₆-alkyl, C₁-C₆-alkyl, C₁-C₆-alkoxy or OH-( C₁-C₆)-alkyl;
R₇ is H, OH or C₁-C₄-alkyl, or R₇ and R₅ form together a bond;
each R₈ is independently OH, C₁-C₆-alkyl, halo-C₁-C₆-alkyl or C₁-C₆-alkoxy;
m is 0, 1, 2 or 3;
n is 0 or 1;
p is 0 or 1;
r is 0 or 1;
t is 0, 1 or 2;
or pharmaceutically acceptable esters or salts thereof.

When X is -CH₂-(CH₂)ₚ- and p is 0, or when X is =NH, 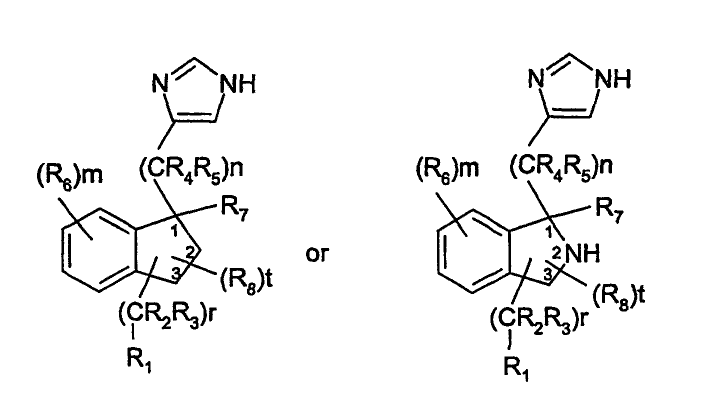 then the bulky substituent -(CR₂R₃)ᵣ-R₁ is preferably at 2- or 3-position of the 5-ring moiety (whereby, of course, in the above formulae the H-atom attached to ring carbon atom or, respectively, ring nitrogen atom will be replaced by the said substituent).

When X is -CH₂-(CH₂)ₚ- and p is 1, 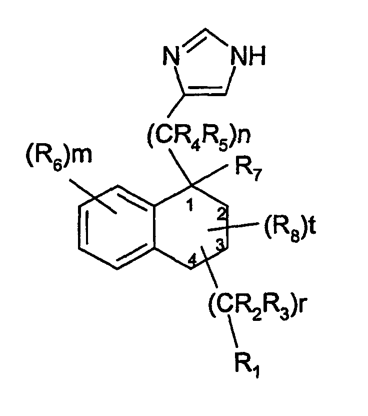 then the bulky substituent -(CR₂R₃)ᵣ-R₁ is preferably at 3- or 4-position of the 6-ring moiety.

The following subgroups (1) to (17) of compounds of formula I taken alone or in any combination with each other are preferred:
1) n is 0;
2) nis1;
3) n is 1 and R₄ and R₅ are H;
4) r is 0;
5) r is 1 and R₂ and R₃ are independently H or C₁-C₄-alkyl;
   preferably H;
6) t is 0;
7) R₇ is H;
8) X is -CH₂-(CH₂)ₚ-; and p is 0 or 1;
9) X is -CH₂-(CH₂)ₚ- and p is 0;
10) X is -CH₂-(CH₂)ₚ- and p is 1;
11) X is -O-;
12) R₁ is phenyl, naphthyl, 1,2,3,4-tetrahydronaphthyl, C₅-C₇-cycloalkyl, C₅-C₇-cycloalkenyl, C₅-C₇-cycloalkynyl, pyridyl, pyrimidinyl, thienyl, furyl, cyclohexyl, piperidyl, piperazinyl or morpholinyl; preferably R₁ is phenyl, naphthyl, pyridyl, thienyl, furyl or cyclohexyl; e.g. R₁ is phenyl; or e.g. R₁ is cyclohexyl;
13) R₁ is as defined above in subgroup (12) substituted with one to three of the substituents selected independently from halogen, - OH, -NH₂, halo-C₁-C₆-alkyl, C₁-C₆-alkyl, C₂-C₆-alkenyl, C₂-C₆-alkynyl and C₁-C₆-alkoxy; prefereably with one to three, e.g. one or two, of the substituents selected independently from halogen, - OH, C₁-C₆-alkoxy and C₁-C₆-alkyl; more preferably from F, -OH and C₁-C₆-alkoxy;
14) m is 0; or m is 1 or 2;
15) m is 1 or 2 and each R₆ is independently halogen, -OH, C₁-C₆-alkoxy or C₁-C₆-alkyl; preferably F, -OH or C₁-C₆-alkoxy;
16) n is 0 and X is -CH₂-(CH₂)ₚ-; and/or
17) n is 1 and X is -CH₂-(CH₂)ₚ-, -O-, =NH or -S-, e.g. -CH₂-(CH₂)ₚ- or -O-.

Preferred subgroups of compounds of formula I are i.a. 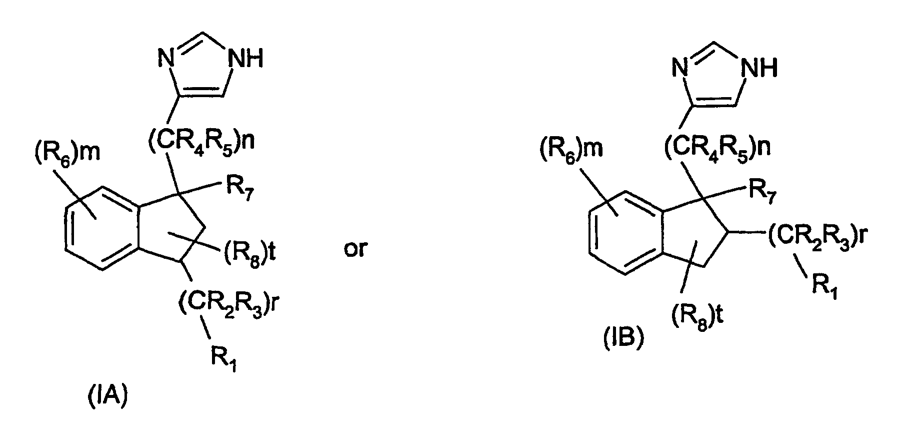 wherein R₁ to R₈, m, n, r and t are as defined above.

In a subgroup of the compounds of formula I, IA or IB, r is 0, or r is 1 and R₂ and R₃ are H. In a further subgroup of the compounds of formula I, IA or IB, n is 0, or n is 1 and R₄, R₅ and R₇ are H. Preferably, t is 0. The optional substituent R₆ is e.g. at 5- and/or 6-position of the indane ring system.

In a further preferred subgroup of the compounds I, IA or IB, R₁ is phenyl, naphthyl, pyridyl, thienyl, furyl or cyclohexyl, e.g. phenyl, pyridyl or cyclohexyl, such as phenyl or cyclohexyl, e.g. phenyl, each of which is optionally substituted with one to three, e.g. one or two, of the substituents selected independently from halogen, -OH, -NH₂, halo-C₁-C₆-alkyl, C₁-C₆-alkyl, C₂-C₆-alkenyl, C₂-C₆-alkynyl, C₁-C₆-alkoxy, OH-(C₁-C₆)-alkyl, NH₂-( C₁-C₆)-alkyl and mono- or di(C₁-C₆-alkyl)amin; e.g. from halogen, -OH, C₁-C₆-alkoxy and C₁-C₆-alkyl; preferably from F, -OH and C₁-C₆-alkoxy.

In a further preferred subgroup of the compounds I, IA or IB m is 0, or m is 1 or 2 and each R₆ is independently halogen, -OH or C₁-C₆-alkoxy.

A further subgroup of the compounds of formula I are compounds of formula IC or ID 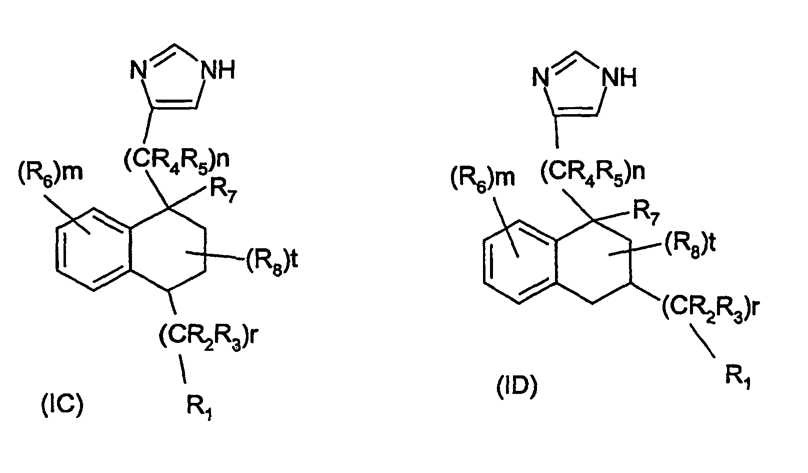 wherein R₁ to R₈, m, n, r and t are as defined above.

A further subgroup of the compounds of formula I are compounds of formula IE 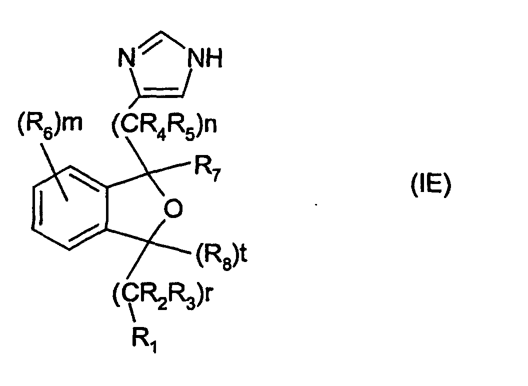 wherein R₁ to R₈, m, n and r are as defined above and t is 0 or 1.

Terms as employed herein have the following meanings: A halogen or halo is e.g. fluorine, chlorine, bromine or iodine, preferably fluorine or chlorine, more preferably fluorine. The term C₁-C₆-alkyl group as employed herein as such or as part of another group includes both straight and branched chain radicals of up to 6 carbon atoms, and preferably of 1 to 4 carbon atoms. The term C₁-C₆-alkoxy refers to -O(C₁-C₆-alkyl) wherein C₁-C₆-alkyl is as defined above. The term C₂-C₆-alkenyl includes both straight and branched chain radicals of up to 6 carbon atoms, preferably of 2 to 4 carbon atoms, containing double bond(s). The term C₂-C₆-alkynyl includes both straight and branched chain radicals of up to 6 carbon atoms, preferably of 2 to 4 carbon atoms, containing triple bond(s). The term halo- C₁-C₆-alkyl refers to C₁-C₆-alkyl radical, as defined above, that is substituted by one or more halo radicals as defined above, e.g. trifluoromethyl, difluoromethyl etc. The term C₃-C₇-cycloalkyl means cyclopropyl, cyclobutyl, cyclopentyl, cyclohexyl or cycloheptyl. C₅-C₇-cycloalkyl means cyclopentyl, cyclohexyl or cycloheptyl, preferably cyclopentyl or cyclohexyl. C₅-C₇-cycloalkenyl or C₅-C₇-cycloalkynyl referes to C₅-C₇-cycloalkyl as defined above, containing double bond(s) or, respectively, a triple bond in its ring structure. Mono- or bicyclic aromatic or partially or fully saturated heterocyclic group from 5 to 10 ring atoms, preferably from 5 to 6 ring atoms, which consists of carbon atoms and one to three, preferably one to two, heteroatoms selected from N, O and/or S, refers e.g. to pyridyl, pyrimidinyl, thienyl, furyl, piperidinyl, piperazinyl or morpholinyl, preferably to pyridyl, thienyl or furyl.

The compounds of formula I and the subgroups IA, IB, IC, ID and IE thereof, as well as the pharmaceutically acceptable esters and salts thereof, are referred to below as the compounds of the invention, unless otherwise indicated.

The compounds of the invention may have chiral carbon atom(s) in their structure. The invention includes within its scope all the possible stereoisomers of the compounds I, including geometric isomers, e.g. Z and E isomers (cis and trans isomers), and optical isomers, e.g. diastereomers and enantiomers. Furthermore, the invention includes in its scope both the individual isomers and any mixtures thereof, e.g. racemic mixtures. The individual isomers may be obtained using the corresponding isomeric forms of the starting material or they may be separated after the preparation of the end compound according to conventional separation methods. For the separation of i.a. optical isomers, e.g. enantiomers, from the mixture thereof the conventional resolution methods, e.g. fractional crystallisation, may be used.

The compounds of the invention can form acid addition salts with both organic and inorganic acids well known in the field of pharmaceuticals. Typical acid addition salts are e.g. chlorides, bromides, sulfates, nitrates, phosphates, sulfonates, formates, tartrates, maleates, citrates, benzoates, salicylates, ascorbates. Furthermore, in the compounds of the invention,
wherein R₆, R₈ and/or the optional substituent at the ring moiety as R₁ is OH, the said -OH functionality may form esters with pharmaceutically acceptable acids which are conventional in the field of pharmaceuticals and which retain the pharmacological properties of the free form. Examples of such esters include esters of aliphatic or aromatic alcohols, e.g. lower alkyl esters, e.g. methyl, ethyl and propyl esters.

The compounds of the invention can be prepared using e.g. the following methods. Accordingly:
(a) The preparation of i.a. compounds of formula I, wherein n is 1, may be illustrated e.g. with the following reaction scheme A: 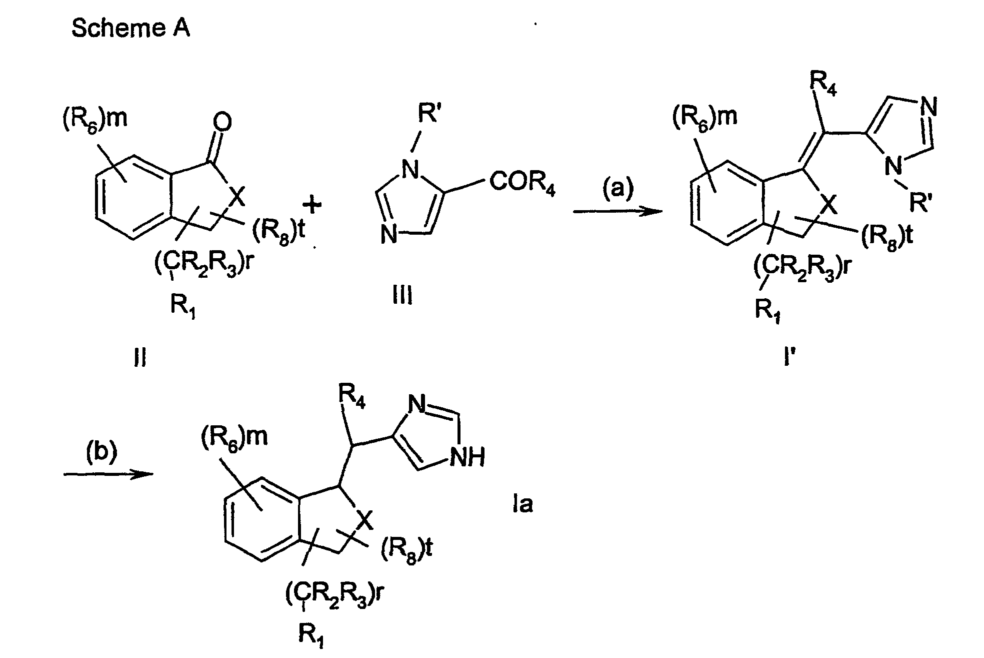 wherein X, R₁ to R₄, R₆, R₈, m, r and t are as defined above and R' is H or a conventional protecting group for =NH in the imidazole ring, e.g. benzyl, trityl (-CPh₃) or SO₂NMe₂.
   The step (a) is a conventional McMurry coupling reaction, i.e. a reductive carbonyl coupling of an imidazole carbaldehyde or an imidazolyl alkylketone III with a ketone II in the presence of a catalyst, e.g. titanium(0) (e.g. produced in situ), in an inert solvent, e.g. THF, at room or elevated temperature. The resulted compound of formula I, wherein R₅ and R₇ form together a bond (I'), may be deprotected, if necessary, and isolated according to the known methods, or converted by hydrogenation of the double bond to another compound of formula I, wherein R₅ and R₇ are H (la, step b). In the hydrogenation step (b) the possible protecting group in the imidazole ring is eliminated simultaneously. The compound of formula I obtained is isolated and worked up in a manner known in the art;
(b) The preparation of i.a. compounds of formula I, wherein n is 0 and R₇ is H, may be illustrated e.g. with the reaction scheme B: 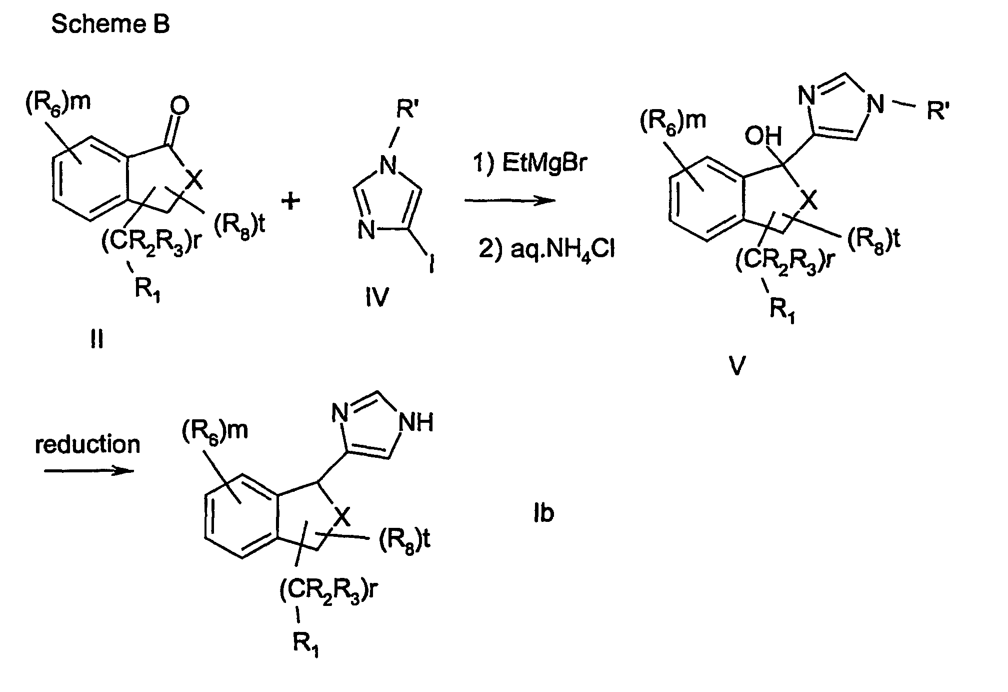 wherein X, R₁ to R₃ , R₆ and R₈ m, r and t are as defined above and R' is a conventional protecting group for =NH in the imidazole ring, e.g. benzyl, trityl (-CPh₃) or SO₂NMe₂.
   In the reaction scheme B, a compound of formula II is first reacted with a compound of formula IV, in the presence of a Grignard-reagent, such as EtMgBr, in a suitable solvent, e.g. CH₂Cl₂, at dry reaction conditions, at room temperature or elevated temperature, and the reaction mixture obtained is then treated with an aqueous NH₄Cl-solution to obtain the compound of formula V. The hydroxyl group and the amino protecting group R' of the compound of formula V can be eliminated in a manner known in the art, e.g. using e.g. TMSCl-Nal-CH₃CN, in a suitable solvent, e.g. CH₂Cl₂, at room or elevated temperature. In the elimination step an intermediate indene-imidazole may be formed, which is further reduced in a manner known in the art. The compound of formula I (Ib) thus obtained is isolated using conventional methods.
(c) The preparation of i.a. compounds of formula I, wherein n is 0, may further be illustrated e.g. with the following reaction scheme C: 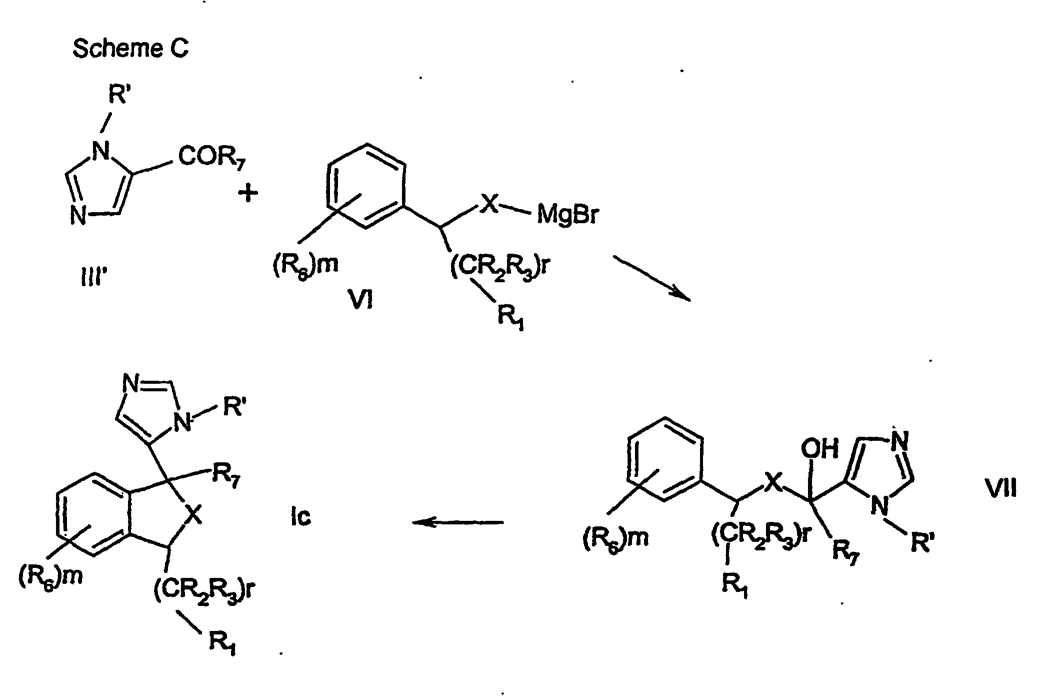
wherein R₁ to R₃, R₆, R₇, m and r are as defined above X is -CH₂-(CH₂)ₚ-, p is 0 or 1 and R' is a conventional protecting group for =NH in the imidazole ring, e.g. benzyl or trityl (-CPh₃).

In the method of scheme C the compound III' is reacted with Grignard reagent VI at room or elevated temperature in a suitable solvent. The resulted compound VII is cyclized in a manner known in the art to obtain the end product Ic.

The other compounds of formula I not illustrated in the above schemes can be prepared according to or analogously to the methods described above or known in the prior art, starting from the suitable starting material. As to the prior art methods reference is made e.g. to WO-A-97 12874, the contents of which are hereby incorporated by reference.

The starting compounds II are commercially available or they may be prepared via a variety of known synthetic routes using suitable starting materials and conventional methods known to those skilled in the art. For instance the compounds of formula II, wherein X is -CH₂-(CH₂)ₚ-, p is 0 or 1, can be prepared according to or analogously to the methods described by Sommer, M.B. et al., J.Org.Chem., vol.55, 1990, p.4822, Welch, W.M. et al., J.Med.Chem., vol.27, 1984, p.1508, and/or Bøgesø, K.P., J.Med.Chem., vol.26, 1983, p.935, the contents of which are hereby incorporated by reference. As a further example, the preparation of compounds II can be carried out according to or analogously to the methods described in the above-mentioned WO-A-97 12874, Miller L.L. and Boyer R.F., J.Am.Chem.Soc., vol.93(3), 1971, p.650-656, or Smonou I. and Orfanopoulos M., Synthetic Communications, vol.20(9), 1990, p.1387-1397.

As to the starting material III, III' and IV, these are commercially available i.a. in an unprotected form, or they may be prepared according to the methods known to those skilled in the art (cf. i.a. Kirk, K.L, J.Heterocycl.Chem., vol.22, 1985, 57). If necessary, the =NH of the imidazole can be protected using conventional methods and protecting groups (R'), e.g. benzyl or trityl. It is understood that, due to the tautomerism, the protecting group R' may be attached to either of the two nitrogen atoms of the imidazole ring.

If necessary, also R₆, R₈ and/or the optional substituent at the ring moiety as R₁ can be protected in a manner known in the art. Such protecting groups as well as the optional protecting group R' can be removed at the final stage using suitable conventional deprotection method(s) known in the art.

It should be noted that the above disclosed synthetic routes are meant to illustrate the preparation of the compounds of the invention and the preparation is by no means limited thereto, i.e. other synthetic processes which are within the general knowledge of a skilled person are also possible.

The compounds of the invention may be converted, if desired, into their pharmaceutically acceptable salt or ester form using methods well known in the art.

As already mentioned hereinbefore, the compounds of the invention show interesting pharmacological properties, namely they exhibit affinity for alpha2 adrenoceptors. The said activity of the compounds of the invention is demonstrated with the pharmacological test presented below.

Antagonist activity on alpha2 adrenoceptors (alpha2AR) in rat *vas deferens in vitro*

Rats were killed by CO₂-suffocation. *Vas deferentia* were dissected out and both prostatic halves were removed to tissue chambers containing Krebs-solution of the following composition (mM): NaCl 118, KCl 4.7, CaCl₂ 2.5, KH₂PO₄ 1.2, MgSO₄ 0.6, NaHCO₃ 25, glucose 11.1, aerated by 5% carbogen, temperature 37°C, pH 7.4. Propranolol 260 g/l and desipramine 2 g/ml were added to prevent the possible effects on alpha-adrenergic receptors and to prevent re-uptake of released norepinephrine, respectively. Preparations were tied to the bottom hooks of the incubation chambers and the to isometric force-displacement transducers above. Electrical stimulation was started after the equilibrium period (5 minutes under a resting tension of 0.5 g) by introducing field stimulation with the following parameters: twin-pulses, voltage 70 V, frequency 0.2 Hz, delay 5 ms, duration 2 ms. As soon as the electrically induced twitch response was stabilised, the test compounds were administered by a cumulative fashion with half logarithmic increments at five minutes intervals. Inhibition of the electrically evoked contractions was measured as the response to alpha2AR agonists. Antagonist was administered into the incubation medium at least five minutes before agonist. Means ± SEM of percentage inhibition were calculated in the absence and in the presence of antagonist and expressed as dose-response curves. In order to express the antagonist potency, pA2-value was calculated.The results of the test are reported in table 1.

**Table 1**

| Compound | *vas deferens* |
|---|---|
| | Alpha2 antagonistic activity |
| Compound 1 | pA2=7.0 |
| Compound 2 | pA2=6.0 |
| Compound 3 | pA2=5.6 |
| Compound 4 | pA2=6.9 |
| Compound 5 | pA2=6.3 |
| Compound 6 | pA2=6.6 |
| Compound 7 | pA2=7.6 |
| Compound 8 | pA2=6.7 |
| Compound 9 | pA2=6.2 |
| Compound 10 | pA2=6.2 |
| Compound 11 | pA2=6.2 |
| Compound 12 | pA2=6.3 |
| Compound 13 | pA2=5.6 |
| Compound 14 | pA2=5.5 |
| Compound 15 | pA2=6.2 |
| Compound 16 | pA2=6.5 |

In general, the compounds of the invention exhibiting alpha2-antagonistic activity may be useful for therapeutical indications in which alpha2-antagonists are used. They may also be used for reversal of the effects of alpha2-agonists.

Accordingly, the compounds of the invention may be useful i.a. in the treatment of different neurological, psychiatric and cognition disorders. Furthermore, they may be used in the treatment of various peripheral disorders, e.g. diabetes, orthostatic hypotension, lipolytic disorders (such as obesity) or sexual dysfunction.

The compounds of the invention may be administered enterally, topically or parenterally.

The compounds of the invention may be formulated alone or together with another active ingredient and/or together with a pharmaceutically acceptable diluent, carrier and/or excipient in different pharmaceutical unit dosage forms, e.g. tablets, capsules, solutions, emulsions and powders etc., depending on the route of adminstration, using conventional techniques. The pharmaceutically acceptable diluent, carrier and/or excipient can be selected from those conventionally used in the field of pharmaceuticals noticing the chosen route of administration.

The amount of the active ingredient varies from 0.01 to 75 weight-% depending on i.a. the type of the dosage form.

The specific dose level of the compounds of the invention depends on several factors such as the compound to be administered, the species, age and the sex of the subject to be treated, the condition to be treated and on the route and method of administration. Accordingly, the dosage for parenteral administration is typically from 0.5 µg/kg to 10 mg/kg per day and that for oral administration is from 5 µg/kg to 100 mg/kg for an adult male.

The present invention also provides a compound of the invention or an ester or salt thereof for use in a method of treatment of human or animal body.

The present invention further provides a compound of the invention or an ester or salt thereof for use in the treatment of different CNS-disorders, such as neurological, psychiatric and cognition disorders, or in the treatment of various peripheral disorders, e.g. diabetes, orthostatic hypotension, lipolytic disorders (such as obesity) or sexual dysfunction.

The invention also provides the use of a compound of the invention or an ester or salt thereof in the manufacture of a medicament for the treatment of different CNS-disorders, e.g. neurological, psychiatric and cognition disorders, or in the treatment of various peripheral disorders, e.g. diabetes, orthostatic hypotension, lipolytic disorders (such as obesity) or sexual dysfunction.

The present invention will be explained in more detail by the following examples. The examples are meant only for illustrating purposes.

### EXAMPLE 1

### 4-(6-Methoxy-3-phenylindan-1-ylmethyl)-1H-imidazole

Titanium(IV)chloride (7.4 ml) was added dropwise to a stirred suspension of zinc powder (8.8 g) in dry tetrahydrofuran (200 ml) with ice cooling under a nitrogen atmosphere. The resulting mixture was heated at reflux for 2 hr with stirring. A solution of 6-methoxy-3-phenyl-1-indanone (4.0 g) and 3-benzyl-3H-imidazole-4-carbaldehyde (4.5 g) in dry tetrahydrofuran (40 ml) was added, and the reflux was continued for 5 hr. The cooled reaction mixture was made alkaline with dilute sodium hydroxide solution. The slurry was filtered, and the filtrate was evaporated to dryness under reduced pressure. The residue was dissolved in acidic water and extracted with dichloromethane. The combined organic phase was washed with water and evaporated to dryness.

The crude intermediate (1-Benzyl-5-(6-methoxy-3-phenylindan-1-ylidenemethyl-1H-imidazole) was dissolved in a solution of ethanol (200 ml), water (20 ml) and hydrochloric acid (1.0 ml). The mixture was hydrogenated at 50-60 °C with 10 % palladium on carbon as catalyst until no more hydrogen was consumed. The mixture was filtered, and the filtrate was evaporated to dryness. The residue was dissolved in water, made alkaline with sodium hydroxide solution and extracted with ethyl acetate. The combined organic phase was washed with water, dried with sodium sulfate and evaporated under reduced pressure to give a crude product of racemic cis and trans diastereoisomers. The product was purified by flash chromatography (elution with a dichloromethane - methanol gradient). The base product was dissolved in ethyl acetate and converted to its hydrochloride salt with hydrogen chloride gas dissolved in ethyl acetate.

¹H NMR (cis isomer as HCl-salt, DMSO-d₆): 1.62-1.70 (m, 1H), 2.50-2.57 (m, 1H), 2.82 (dd, J=14.7 Hz, J=9.5 Hz, 1H), 3.45 (dd, J=14.7 Hz, J=4.6 Hz, 1H), 3.48-3.56 (m, 1 H), 3.75 (s, 3H), 4.16-4.21 (m, 1H), 6.67-7.34 (m, 8H), 7.46 (s, 1H), 9.03 (s, 1H)

¹H NMR (trans isomer as HCl-salt, DMSO-d₆): 2.08-2.17 (m, 1H), 2.23-2.31 (m, 1H), 2.84 (dd, J=14.7 Hz, J=9.5 Hz, 1H), 3.12 (dd, J=14.7 Hz, J=4.6 Hz, 1H), 3.60-3.69 (m, 1H), 3.72 (s, 3H), 4.32-4.39 (m, 1H), 6.75-7.35 (m, 8H), 7.43 (s, 1H), 8.99 (s, 1H)

Using the same method the following compounds were prepared:

### 4-(3-Phenylindan-1-ylmethyl)-1H-imidazole (compound 1)

¹H NMR (cis isomer as HCl-salt, DMSO-d₆): 1.64-1.72 (m, 1H), 2.51-2.58 (m, 1H), 2.82 (dd, J=15.0 Hz, J=9.6 Hz, 1H), 3.45 (dd, J=15.0 Hz, J=4.3 Hz, 1H), 3.49-3.57 (m, 1H), 4.24-4.29 (m, 1H), 6.79 (d, J=7.5 Hz, 1H), 7.16-7.35 (m, 8H), 7.47 (d, J=1.3 Hz, 1H), 8.99 (d, J=1.3 Hz, 1 H)

### 4-(5,6-Dimethoxy-3-phenylindan-1-ylmethyl)-1H-imidazole (compound 2)

¹H NMR (cis isomer as HCl-salt, MeOH-d₄): 1.61-1.71 (m, 1H), 2.62-2.72 (m, 1H), 2.91 (dd, J=15.1 Hz, J=9.1 Hz, 1H), 3.43 (dd, J=15.1Hz, J=4.6 Hz, 1H), 3.49-3.55 (m, 1H), 3.66 (s, 3H), 3.85 (s, 3H), 4.21-4.25 (m, 1H), 6.43 (s, 1H), 6.92 (s, 1H), 7.10-7.33 (m, 6H), 8.79 (d, J=1.1 Hz, 1H)

### 4-[6-Methoxy-3-(4-methoxyphenyl)indan-1-ylmethyl]-1H-imidazole (compound 5)

¹H NMR (cis isomer as HCl-salt, DMSO-d₆): 1.57-1.65 (m, 1H), 2.46-2.54 (m, 1H), 2.80 (dd, J=14.8 Hz, J=9.4 Hz, 1H), 3.44 (dd, J=14.8 Hz, J=4.6 Hz, 1H), 3.46-3.54 (m, 1H), 3.73 (s, 3H), 3.75 (s, 3H), 4.10-4.14 (m, 1H), 6.67 (d, J=8.3 Hz, 1H), 6.74 (dd, J=8.3 Hz, J=2.2 Hz, 1H), 6.85-6.90 (m, 3H), 7.06-7.10 (m, 2H), 7.47 (s, 1H), 9.02 (s, 1H)

### 4-[3-(4-Methoxyphenyl)indan-1-ylmethyl]-1H-imidazole

¹H NMR (cis isomer as HCl-salt, DMSO-d₆): 1.59-1.70 (m, 1H), 2.46-2.55 (m, 1H), 2.83 (dd, J=14.7 Hz, J=9.5 Hz, 1H), 3.45 (dd, J=14.7 Hz, J=4.7 Hz, 1H), 3.49-3.57 (m, 1H), 3.74 (s, 3H), 4.16-4.22 (m, 1H), 6.78 (d, J=7.3 Hz, 1H), 6.86-6.91 (m, 2H), 7.07-7.12 (m, 2H), 7.13-7.34 (m, 3H), 7.46 (d, J=1.4 Hz, 1H), 9.05 (d, J=1.4 Hz, 1H)

### 4-[3-(4-Fluorophenyl)indan-1-ylmethyl]-1H-imidazole (compound 7)

¹H NMR (cis isomer as HCl-salt, DMSO-d₆): 1.62-1.71 (m, 1H), 2.50-2.57 (m, 1H), 2.83 (dd, J=14.8 Hz, J=9.7 Hz, 1H), 3.46 (dd, J=14.8 Hz, J=4.5 Hz, 1H), 3.53-3.60 (m, 1H), 4.26-4.31 (m, 1H), 6.78 (d, J=7.4 Hz, 1H), 7.13-7.35 (m, 7H), 7.47 (d, J=1.3 Hz, 1H), 9.05 (d, J=1.3 Hz, 1H)

### 4-[3-(3-Fluorophenyl)indan-1-ylmethyl]-1H-imidazole (compound 9)

¹H NMR (cis isomer as HCl-salt, DMSO-d₆): 1.67-1.76 (m, 1H), 2.51-2.58 (m, 1H), 2.85 (dd, J=14.9 Hz, J=9.7 Hz, 1 H), 3.46 (dd, J=14.9 Hz, J=4.5 Hz, 1 H), 3.53-3.59 (m, 1H), 4.29-4.34 (m, 1H), 6.81 (d, J=7.4 Hz, 1H), 6.99-7.41 (m, 7H), 7.47 (d, J=1.2 Hz, 1H), 9.05 (d, J=1.2 Hz, 1H)

### 4-[3-(2-Fluorophenyl)indan-1-ylmethyl]-1H-imidazole (compound 10)

¹H NMR (cis isomer as HCl-salt, DMSO-d₆): 1.71-1.79 (m, 1H), 2.65-2.72 (m, 1H), 2.95 (dd, J=15.1 Hz, J=9.2 Hz, 1H), 3.43 (dd, J=15.1 Hz, J=4.8 Hz, 1H), 3.58-3.65 (m, 1H), 4.58-4.62 (m, 1H), 6.89 (d, J=7.3 Hz, 1H), 7.06-7.34 (m, 8H), 8.79 (d, J=1.3 Hz, 1H)

### 4-[3-(3,4-Difluorophenyl)indan-1-ylmethyl]-1H-imidazole (compound 11)

¹H NMR (cis isomer as HCl-salt, DMSO-d₆): 1.66-1.75 (m, 1H), 2.50-2.57 (m, 1H), 2.84 (dd, J=14.9 Hz, J=9.7 Hz, 1H), 3.46 (dd, J=14.9 Hz, J=4.4 Hz, 1H), 3.52-3.58 (m, 1H), 4.29-4.33 (m, 1H), 6.81 (d, J=7.4 Hz, 1H), 7.05-7.43 (m, 6H), 7.48 (d, J=1.3 Hz, 1H), 9.05 (d, J=1.3 Hz, 1H)

### 4-[6-Fluoro-3-(4-fluorophenyl)indan-1-ylmethyl]-1H-imidazole (compound 15)

¹H NMR (cis isomer as HCl-salt, DMSO-d₆): 1.65-1.76 (m, 1H), 2.52-2.61 (m, 1H), 2.84 (dd, J=14.8 Hz, J=9.5 Hz, 1H), 3.44 (dd, J=14.8 Hz, J=4.6 Hz, 1H), 3.50-3.56 (m, 1H), 4.23-4.30 (m, 1H), 6.76-7.25 (m, 7H), 7.47 (d, J=1.2 Hz, 1H), 9.00 (d, J=1.2 Hz, 1H)

### 4-[3-(4-Fluorophenyl)-6-methoxyindan-1-ylmethyl]-1H-imidazole

¹H NMR (cis isomer as HCl-salt, DMSO-d₆): 1.62-1.70 (m, 1H), 2.62-2.69 (m, 1H), 2.91 (dd, J=14.9 Hz, J=9.1 Hz, 1H), 3.46 (dd, J=14.9 Hz, J=4.8 Hz, 1H), 3.52-3.57 (m, 1H), 3.79 (s, 3H), 4.20-4.25 (m, 1H), 6.72-7.20 (m, 7H), 7.34 (d, J=1.3 Hz, 1H), 8.83 (d, J=1.3 Hz, 1H)

### 4-[3-(4-Fluorophenyl)-6-trifluoromethylindan-1-ylmethyl]-1H-imidazole

¹H NMR (cis-isomer as HCl-salt, MeOH-d₄): 1.74-1.83 (m, 1H), 2.70-2.77 (m, 1H), 2.95 (dd, J=14.9 Hz, J=9.5 Hz, 1H), 3.57 (dd, J=14.9 Hz, J=4.7 Hz, 1H), 3.60-3.65 (m, 1H), 4.36-4.40 (m, 1H), 7.02-7.09 (m, 3H), 7.18-7.23 (m, 2H), 7.39 (d, J=1.1 Hz, 1H), 7.50 (d, J=8.0 Hz, 1H), 7.63 (s, 1H), 8.85 (s, 1H)

### 4-(6-Fluoro-3-phenylindan-1-ylmethyl)-1H-imidazole

¹H NMR (cis-isomer as HCl-salt, DMSO-d₆): 1.69-1.78 (m, 1H), 2.53-2.60 (m, 1H), 2.85 (dd, J=14.9 Hz, J=9.7 Hz, 1H), 3.47 (dd, J=14.9, J=4.3 Hz, 1H), 3.56-3.63 (m, 1H), 4.21-4.26 (m, 1H), 6.76-7.35 (m, 8H), 7.48 (s,1H), 9.06 (s, 1H)

¹H NMR (trans-isomer as HCl-salt, DMSO-d₆): 2.14-2.21 (m, 1H), 2.28-2.35 (m, 1H), 2.86 (dd, J=14.8 Hz, J=9.2 Hz, 1H), 3.14 ((dd, J=14.9 Hz, J=5.2 Hz, 1H), 3.68-3.75 (m, 1H), 4.42-4.46 (m, 1H), 6.92-7.32 (m, 8H), 7.45 (s, 1H), 9.06 (s, 1H)

### EXAMPLE 2

### 4-(3-Phenyl-1,3-dihydroisobenzofuran-1-ylmethyl)-1H-imidazole (compound 12)

This compound was prepared according to the procedure of Example 1 except that 3-phenylphthalide was used in place of 3-phenyl-1-indanone.

¹H NMR (cis isomer as HCl-salt, DMSO-d₆): 3.22 (dd, J=15.4 Hz, J=7.4 Hz, 1H), 3.46 (dd, J=15.4 Hz, J=4.1 Hz, 1H), 5.56-5.60 (m, 1H), 6.10 (s, 1H), 6.94 (d, J=7.4 Hz, 1H), 7.20-7.39 (m, 9H), 9.00 (d, J=1.3 Hz, 1H)

### EXAMPLE 3

### 4-(4-Phenyl-1,2,3,4-tetrahydronaphthalen-1-ylmethyl)-1H-imidazole

This compound was prepared according to the procedure of Example 1 except that 4-phenyl-1-tetralone was used in place of 3-phenyl-1-indanone.

¹H NMR (cis-isomer as HCl-salt, DMSO-d₆): 1.51-1.57 (m, 1H), 1.70-1.78 (m, 1H), 1.91-1.96 (m, 2H), 3.03 (dd, J=14.8 Hz, J=10.2 Hz, 1H), 3.12 (dd, J=14.8 Hz, J=4.4 Hz, 1 H), 3.24-3-31 (m, 1H), 4.04-4.08 (m, 1H), 6.70 (d, J=7.1 Hz, 1H), 7.00-7.45 (m, 9H), 9.06 (d, J=1.3 Hz, 1H)

### EXAMPLE 4

### 3-(1H-imidazol-4-ylmethyl)-1-phenylindan-5-ol (compound 4)

A mixture of 4-(6-methoxy-3-phenylindan-1-ylmethyl)-1H-imidazole (500 mg) and 48 % hydrobromic acid (20 ml) was heated at reflux for 1 hr with stirring. The cooled reaction mixture was poured into water and made basic with ammonium hydroxide solution. The resulting precipitate was filtered and washed with water. The product was purified by flash chromatography (elution with a dichloromethane - methanol gradient).

¹H NMR (cis isomer, MeOH-d₄): 1.62-1.71 (m. 1H), 2.54-2.61 (m, 1H), 2.74 (dd, J=14.6 Hz, J=8.8 Hz, 1H), 3.22 (dd, J=14.6 Hz, J=5.2 Hz, 1H), 3.40-3.49 (m, 1H), 4.09-4.14 (m, 1H), 6.55-6.77 (m, 4H), 7.12-7.28 (m, 5H), 7.57 (d, J=1.1 Hz, 1H)

¹H NMR (trans isomer, MeOH-d₄): 2.09-2.16 (m. 1H), 2.29-2.35 (m, 1H), 2.74 (dd, J=14.6 Hz, J=8.8 Hz, 1H), 2.94 (dd, J=14.6 Hz, J=5.4 Hz, 1H), 3.50-3.58 (m, 1H), 4.23-4.28 (m, 1H), 6.53-6.73 (m, 4H), 7.07-7.25 (m, 5H), 7.58 (s, 1H)

Using the same method the following compounds were prepared:

### 4-[3-(1H-Imidazol-4-ylmethyl)indan-1-yl]phenol

¹H NMR (cis isomer as HCl-salt, DMSO-d₆): 1.58-1.66 (m, 1H), 2.45-2.50 (m, 1H), 2.80 (dd, J=14.7 Hz, J=9.5 Hz, 1H), 3.44 (dd, J=14.7 Hz, J=4.6 Hz, 1H), 3.46-3.52 (m, 1H), 4.11-4.16 (m, 1H), 6.70-6.74 (m, 2H), 6.78 (d, J=7.4 Hz, 1H), 6.95-6.99 (m, 2H), 7.14-7.23 (m, 2H), 7.31 (d, J=7.4 Hz, 1H), 7.47 (d, J=1.3 Hz, 1H), 9.03 (d, J=1.3 Hz, 1H), 9.30 (s, 1H)

### 1-(1H-imidazol-4-ylmethyl)-3-phenylindan-5,6-diol (compound 3)

¹H NMR (cis isomer as HCl-salt, MeOH-d₄): 1.56-1.66 (m. 1H), 2.57-2.66 (m, 1H), 2.87 (dd, J=15.0 Hz, J=8.9 Hz, 1H), 3.31 (dd, J=15.0 Hz, J=4.9 Hz, 1H), 3.39-3.46 (m, 1 H), 4.12-4.17 (m, 1H), 6.28 (s, 1H), 6.69 (s, 1H), 7.13-7.30 (m, 6H), 8.79 (s, 1H)

### 1-(4-Hydroxyphenyl)-3-(1H-imidazol-4-ylmethyl)indan-5-ol (compound 6)

¹H NMR (cis isomer as HCl-salt, DMSO-d₆): 1.52-1.60 (m. 1H), 2.41-2.48 (m, 1H), 2.75 (dd, J=14.7 Hz, J=9.4 Hz, 1H), 3.32 (dd, J=14.7 Hz, J=5.1 Hz, 1H), 3.36-3.43 (m, 1H), 3.99-4.03 (m, 1H), 6.57-6.98 (m, 7H), 7.45 (d, 1.3 Hz, 1H), 9.01 (d, 1.3 Hz, 1H), 9.22 (s, 1 H), 9.24 (s, 1H)

### 1-(4-Fluorophenyl)-3-(1H-imidazol-4-ylmethyl)indan-5-ol (compound 8)

¹H NMR (cis isomer as HCl-salt, DMSO-d₆): 1.56-1.64 (m, 1H), 2.47-2.54 (m, 1H), 2.77 (dd, J=14.9 Hz, J=9.6 Hz, 1H), 3.34 (dd, J=14.9 Hz, J=5.0 Hz, 1H), 3.40-3.46 (m, 1H), 4.14-4.18 (m, 1H), 6.55-6.69 (m, 3H), 7.10-7.23 (m, 4H), 7.47 (d, 1.3 Hz, 1H), 9.04 (d, J=1.3 Hz, 1H), 9.33 (s, 1H)

### EXAMPLE 5

### 4-(2-Benzylindan-1-ylmethyl)-1H-imidazole

### a) 2-Benzylideneindan-1-one

To a solution of 1-indanone (5.0 g) and benzaldehyde (4.1 g) in methanol (40 ml), was added 2.2 ml of 48 % aqueous sodium hydroxide solution. The reaction mixture was stirred at room temperature for 0.5 hr. The resulting precipitate was filtered and washed with water. The yield was 7.9 g.

¹H NMR (DMSO-d₆): 4.14 (s, 2H), 7.47-7.81 (m, 10H)

### b) 2-Benzylindan-1-one

2-Benzylideneindan-1-one (6.0 g) was hydrogenated in 100 ml of ethanol using 0.1 g of 10 % palladium on carbon as catalyst at room temperature. The catalyst was removed by filtration, and the filtrate was evaporated to dryness.

¹H NMR (DMSO-d₆): 2.67-2.72 (m, 1H), 2.78-2.83 (m, 1H), 3.01-3.08 (m, 1 H), 3.10-3.21 (m, 2H), 7.17-7.68 (m, 9H)

### c) 4-(2-Benzylindan-1-ylmethyl)-1H-imidazole

This compound was prepared according to the procedure of Example 1 except that 2-benzylindan-1-one was used in place of 3-phenyl-1-indanone.

¹H NMR (cis isomer as HCl-salt, DMSO-d₆): 2.47-2.53 (m, 1H), 2.63-2.67 (m, 2H), 2.74-2.82 (m, 1H), 2.86-2.93 (m, 2H), 3.02-3.07 (m, 1H), 3.59-3.65 (m, 1H), 6.75 (d, J=7.4 Hz, 1H), 7.02-7.32 (m, 8H), 7.39 (d, J=1.2 Hz, 1H), 9.07 (d, J=1.2 Hz, 1H)

### EXAMPLE 6

### 4-[(2,3-Dihydro-6-methoxy-2-phenyl-1H-inden-1-yl)methyl]-1H-imidazole (compound 13) and 3-(1H-Imidazol-4-ylmethyl)-2-phenylindan-5-ol (compound 14)

### (a) 3-(4-Methoxyphenyl)-2-phenylacrylic acid

A mixture of 4-methoxybenzaldehyde (30.0 g, 0.22 mol), phenylacetic acid (31.5 g, 0.23 mol), and triethylamine (31 ml) in acetic anhydride (75 ml) was heated at 90 °C for 5 hours. After cooling, 18 ml of water was dropped carefully during 15 min. Then, potassium carbonate (243 g) in water (1800 ml) was dropped and the solution was heated at 60 °C for 1 hour. After cooling, the solution was extracted with dichloro-methane. When the aqueous phase was acidified (pH 6-7), the product was precipitated. After stirring at 0 °C, the product was filtered and dried.

¹H NMR (CDCl₃): δ 3.75 (3H, s), 6.68 (2H, d, ³J = 8.8 Hz), 6.99 (2H, d, ³J = 9.0 Hz), 7.23 - 7.26 (2H, m), 7.35 - 7.42 (3H, m), 7.84 (1H, s)

### (b) 3-(4-Methoxyphenyl)-2-phenylpropanoic acid

Palladium on activated carbon (10 % wt., 2.77 g) was added to a solution of 3-(4-methoxyphenyl)-2-phenylacrylic acid (27.7 g, 0.11 mol) in acetic acid (1000 ml). The mixture was hydrogenated at ambient temperature. The mixture was filtered through Celite, and the solvent was evaporated. The product was recrystallized from a small amount of ethyl acetate. Melting point 220-221 °C.

¹H NMR (CDCl₃): δ 2.84 (1H, dd, ²J_{gem} = 13.8 Hz, ³J = 9.5 Hz), 3.23 (1H, dd, ²J_{gem} = 13.9 Hz, ³J = 5.9 Hz), 3.48 (1H, dd, ³J = 9.4 Hz, ³J = 6.1 Hz), 3.67 (3H, s), 6.65 (2H, d, ³J = 8.7 Hz), 6.87 (2H, d, ³J = 8.6 Hz), 7.04 - 7.14 (5H, m)

### (c) 3-(4-Methoxyphenyl)-2-phenylpropionyl chloride

3-(4-Methoxyphenyl)-2-phenylpropanoic acid (12.5 g, 0.049 mol) was converted to its acid chloride by treatment with thionyl chloride (8.2 ml) in dry dichloromethane (75 ml) at 40 °C. Excess thionyl chloride and dichloromethane were evaporated off. The crude product was used in the next step without purification.

¹H NMR (CDCl₃): δ 3.02 (1H, dd, ²J_{gem} = 14.1 Hz, ³J = 7.2 Hz), 3.43 (1H, dd, ²J_{gem} =14.1 Hz, ³J = 7.9 Hz), 3.76 (3H, s), 4.22 (1H, t, ³J = 7.5 Hz), 6.77 (2H, d, ³J = 8.7 Hz), 6.99 (2H, d, ³J = 8.7 Hz), 7.23 - 7.26 (2H, m), 7.30 - 7.38 (3H, m)

### (d) 6-Methoxy-2-phenylindan-1-one

Aluminum chloride (345 mg) and one quarter of the crude 3-(4-methoxyphenyl)-2-phenylpropionyl chloride from the previous step were added to dry dichloromethane (45 ml) at 0 °C. After 1 hour aluminum chloride (345 mg) and the second quarter of the acid chloride were added. Stirring was continued and the addition was repeated twice again. After the last addition strirring was continued for half an hour at 0 °C and then 2 hours at room temperature. The reaction mixture was poured into icecold diluted acidic water. The organic phase was separated and the water phase was extracted twice with dichloromethane. The combined organic phases were washed with water, 2.5 % sodium hydroxide solution in water and again with water. The dichloromethane solution was dried and evaporated.

¹H NMR (CDCl₃): δ 3.19 (1H, dd, ²J_{gem} = 17.1 Hz, ³J = 3.8 Hz), 3.62 (1H, dd, ²J_{gem} = 17.1 Hz, ³J = 8.1 Hz), 3.85 (3H, s), 3.92 (1H, dd, ³J = 8.1 Hz, ³J = 3.8 Hz), 7.17 - 7.19 (2H, m), 7.23 - 7.27 (3H, m), 7.30 - 7.34 (2H, m), 7.40 - 7.43 (1H, m)

### (e) 1-Benzyl-5-(6-methoxy-2-phenylindan-1-ylidenemethyl)-1H-imidazole

Titanium(IV)chloride (13.2 ml, 22.8 g, 0.12 mol) was added dropwise to a stirred suspension of activated zinc powder (15.9 g, 0.24 mol) in dry tetrahydrofuran (240 ml) at -5 °C - (-10 °C) under a nitrogen atmosphere. After completion of the addition the resulting mixture was refluxed for 2 hours with stirring. A solution of 6-methoxy-2-phenylindan-1-one (7.54 g, 0.032 mol) and 3-benzyl-3H-imidazole-4-carbaldehyde (7.37 g, 0.040 mol) in dry tetrahydrofuran (110 ml) was added dropwise to a refluxing mixture. After the addition the mixture was refluxed for 5 hours. Then the mixture was cooled to 50 °C and 50 ml of methanol and 25 ml of water was added, respectively. The cooled reaction mixture was made alkaline (pH 8-9) with 50 % sodium hydroxide solution in water. The slurry was filtered through Celite, and the filtrate was evaporated to dryness under reduced pressure. Ethyl acetate (100ml) was added to the residue and the mixture was heated. The cooled mixture was filtered and the filtrate was washed with water. The organic phase was dried and evaporated to dryness. The crude product was used in the next step.

### (f) 4-[(2,3-Dihydro-6-methoxy-2-phenyl-1H-inden-1-yl)methyl]-1H-imidazole (compound 13)

A solution of the crude 1-benzyl-5-(6-methoxy-2-phenylindan-1-ylidenemethyl)-1H-imidazole (1.00 g) in acetic acid (100 ml) was shaken with 10 % palladium on charcoal (100 mg) for 7 hours at 80 °C under 3 atm of hydrogen on a Parr hydrogenator. The mixture was filtered through Celite, and the filtrate was evaporated to dryness. The residue was dissolved in water, made alkaline (pH 9) and extracted with ethyl acetate. The combined organic phases were washed with water, dried with sodium sulfate and evaporated under reduced pressure to give a crude product which is the mixture of cis and trans diastereomers of 4-[(2,3-dihydro-6-methoxy-2-phenyl-1H-inden-1-yl)methyl]-1H-imidazole. The product was purified by flash chromatography (elution with a dichloromethane - methanol gradient). The base product was converted to its hydrochloride salt by dissolving the base in ethyl acetate and adding hydrogen chloride in ethyl acetate. The product is the mixture of the diastereomers (cis:trans 94:6, mp. 158-159 °C).

The cis diastereomer as its hydrochloride salt: ¹H NMR (CD₃OD): δ 2.52 (1H, distorted ddd, ²J_{gem} = 15.2 Hz, ³J = 7.4 Hz, ⁴J = 0.8 Hz), 2.67 (1H, distorted ddd, ²J_{gem} = 15.4 Hz, ³J = 8.0 Hz, ⁴J = 0.6 Hz), 3.16 (1H, distorted dd, ²J_{gem} = 15.4 Hz, ³J = 7.6 Hz), 3.27 - 3.33 (1H, m), 3.71 (3H, s), 3.76 (1H, m), 3.90 (1H, m), 6.39 (1H, d, Jₘₑₜₐ = 2.4 Hz), 6.79 (1H, dd, Jₒᵣₜₒ = 8.2 Hz, Jₘₑₜₐ = 2.5 Hz), 6.96 (1H, d, ⁴J = 1.2 Hz), 7.18 - 7.29 (6H, m), 8.68 (1H, d, ⁴J = 1.4 Hz)

### (g) 3-(1H-Imidazol-4-ylmethyl)-2-phenylindan-5-ol (compound 14)

A mixture of 4-[(2,3-dihydro-6-methoxy-2-phenyl-1H-inden-1-yl)methyl]-1 H-imidazole (370 mg as base) and 48 wt. % hydrobromic acid (15 ml) was heated at 130-140 °C for 2 hours with stirring. The cooled reaction mixture was poured into water and made basic (pH 8). The resulting precipitate was filtered and washed with water. The product was purified by flash chromatography (elution with a dichloromethane - methanol gradient).

The cis diastereomer: ¹H NMR (CD₃OD): δ 2.37 (1 H, distorted ddd, ²J_{gem} = 14.7 Hz, ³J = 6.2 Hz, ⁴J = 0.8 Hz), 2.44 (1H, distorted ddd, ²J_{gem} = 14.7 Hz, ³J = 8.9 Hz), 3.10 (1H, distorted dd, ²J_{gem} = 15.0 Hz, ³J = 7.3 Hz), 3.16 (1H, distorted dd, ²J_{gem} = 15.1 Hz, ³J = 7.4 Hz), 3.68 - 3.80 (2H, m), 6.23 (1H, d, Jₘₑₜₐ = 2.3 Hz), 6.52 (1H, s), 6.60 (1H, dd, Jₒᵣₜₒ = 8.1 Hz, Jₘₑₜₐ = 2.4 Hz), 7.06 (1H, d, Jₒᵣₜₒ = 8.1 Hz), 7.14 - 7.19 (3H, m), 7.21 - 7.25 (2H, m), 7.63 (1H, d, ⁴J = 1.0 Hz)

The trans diastereomer: ¹H NMR (CD₃OD): δ 2.84 -2.91 (2H, m), 3.04 (1H, distorted dd, ²J_{gem} = 14.9 Hz, ³J = 6.4 Hz), 3.16 - 3.23 (1H, m), 3.26 - 3.29 (1H, m), 3.58 (1H, m), 6.55 (1H, d, Jₘₑₜₐ = 2.2 Hz), 6.63 (1H, dd, Jₒᵣₜₒ = 8.6 Hz, Jₘₑₜₐ = 2.3 Hz), 6.79 (1H, s), 7.01 (1H, d, Jₒᵣₜₒ = 8.1 Hz), 7.07 - 7.13 (3H, m), 7.15 - 7.21 (2H, m), 7.79 (1H, s)

### EXAMPLE 7

### 4-[(2,3-Dihydro-2-phenyl-1H-inden-1-yl)methyl]-1H-imidazole (compound 16)

### (a) 2,3-Diphenylpropanoic acid

10 % Palladium on charcoal (0.8 g) was added to a solution of a-phenylcinnamic acid (10.0 g, 0.0445 mol) in ethanol (200 ml). The mixture was hydrogenated at ambient temperature. The mixture was filtered through Celite, and the solvent was evaporated.

¹H NMR (DMSO-d₆): δ 2.94 (1H, dd, ²J_{gem} = 13.8 Hz, ³J = 6.9 Hz), 3.29 (1H, dd, ²J_{gem} =13.8 Hz, ³J = 8.5 Hz), 3.86 (1H, dd, ³J = 8.7 Hz, ³J = 6.9 Hz), 7.12 - 7.25 (6H, m), 7.28 - 7.34 (4H, m)

### (b) 2-Phenylindan-1-one

Polyphosphoric acid (50 g) was heated in an oil bath at 140-145 °C and 2,3-diphenyl-propanoic acid (2.5 g) was added. Heating was continued for 45 min. Water was added. The mixture was cooled and extracted with ethyl acetate. The organic extracts were washed with 1 M NaOH solution and water. After drying the solvent was evaporated under reduced pressure. The product thus obtained was further purified by trituration in heptane.

¹H NMR (DMSO-d₆): δ 3.21 (1H, dd, ²J_{gem} = 17.4 Hz, ³J = 4.2 Hz), 3.69 (1 H, dd, ²J_{gem} =17.6 Hz, ³J = 8.3 Hz), 4.01 (1H, dd, ³J = 8.3 Hz, ³J = 4.2 Hz), 7.16 - 7.19 (2H, m), 7.22 - 7.27 (1H, m), 7.30 - 7.35 (2H, m), 7.47 - 7.51 (1H, m), 7.65 - 7.77 (3H, m)

### (c)1-Benzyl-5-(2-phenylindan-1-ylidenemethyl)-1H-imidazole

1-Benzyl-5-(2-phenylindan-1-ylidenemethyl)-1H-imidazole was prepared as 1-benzyl-5-(6-methoxy-2-phenylindan-1-ylidenemethyl)-1H-imidazole above except that 2-phen-ylindan-1-one was used as a starting material. In this case after evaporation of the filtrate the residue was dissolved in diluted hydrochloric acid. The product was extracted into dichloromethane. The combined organic phases were washed with water, dried over sodium sulfate and evaporated under reduced pressure. The crude product as its hydrochloride salt was used in the next step without purification.

### (d) 4-[(2,3-Dihydro-2-phenyl-1H-inden-1-yl)methyl]-1H-imidazole

The crude 1-benzyl-5-(2-phenylindan-1-ylidenemethyl)-1H-imidazole in acetic acid was shaken with palladium on charcoal as 1-benzyl-5-(6-methoxy-2-phenylindan-1-ylidenemethyl)-1H-imidazole above except in this case for 2 days at 60-70 °C under normal pressure. The hydrochloride salt of the purified product was the mixture of the cis and trans diastereomers (cis:trans 96:4).

The cis diastereomer as its hydrochloride salt: ¹H NMR (DMSO-d₆): δ 2.36 (1H, distorted dd, ²J_{gem} = 15.0 Hz, ³J = 5.2 Hz), 2.55 (1H, distorted dd, ²J_{gem} = 15.1 Hz, ³J = 8.8 Hz), 3.19 (1H, distorted dd, ²J_{gem} = 15.6 Hz, ³J = 7.2 Hz), 3.28 - 3.36 (2H, m), 3.87 s(2H, m), 6.73 (1H, d, Jₒᵣₜₒ = 7.4 Hz), 7.06 - 7.11 (2H,m), 7.18 - 7.36 (7H, m), 8.95 (1H, d, ⁴J = 1.3 Hz)

### EXAMPLE 8

### 4-[(3-Cyclohexyl-2,3-dihydro-1H-inden-1-yl)methyl]-1H-imidazole

### (a) 3-Cyclohexylindan-1-one

This compound was prepared according to the method described by B. M. Trost and L. H. Latimer in J. Org. Chem. 42 (1977) 3212. The starting compounds were 1-indanone and cyclohexyl bromide.

¹H NMR (CDCl₃): δ 0.83 - 1,35 (6H, m), 1.65 - 1.90 (5H, m), 2.51 (1H, dd, ²J_{gem} = 19.1 Hz, ³J = 3.0 Hz), 2.67 (1H, dd, ²J_{gem} = 19.1 Hz, ³J = 7.8 Hz), 3.38 (1H, m), 7.36 (1H, m), 7.49 (1H, m), 7.59 (1H, m), 7.73 (1H, m)

### (b) 1-Benzyl-5-(3-cyclohexylindan-1-ylidenemethyl)-1H-imidazole

1-Benzyl-5-(3-cyclohexylindan-1-ylidenemethyl)-1H-imidazole was prepared as 1-benzyl-5-(2-phenylindan-1-ylidenemethyl)-1H-imidazole above except that 3-cyclo-hexylindan-1-one was used as a starting material. The crude product as its hydro-chloride salt was used in the next step without purification.

### (c) 4-[(3-Cyclohexyl-2,3-dihydro-1H-inden-1-yl)methyl]-1H-imidazole

4-[(3-Cyclohexyl-2,3-dihydro-1H-inden-1-yl)methyl]-1H-imidazole was prepared as 4-[(2,3-dihydro-6-methoxy-2-phenyl-1H-inden-1-yl)methyl]-1H-imidazole above except that 1-benzyl-5-(3-cyclohexylindan-1-ylidenemethyl)-1 H-imidazole was used as a starting material. The crude product which is the mixture of cis and trans diastereomers of 4-[(3-cyclohexyl-2,3-dihydro-1H-inden-1-yl)methyl]-1H-imidazole was.purified by flash chromatography (eluent: 9.75:0.25 (v/v) mixture of dichloromethane―methanol). The free base was converted to its hydrochloride salt which was the mixture of the diastereomers (cis:trans 98:2).

The cis diastereomer as its hydrochloride salt: ¹H NMR (CD₃OD): δ 0.92 (1H, m), 1.10 - 1.43 (5H, m), 1.46 (1H, dt, ³J = 12.4 Hz, ³J = 9.5 Hz), 1.67 -1.84 (4H, m) 1.92 (1H, m), 2.18 (1H, dt, ³J = 12.4 Hz, ³J = 7.7 Hz), 2.86 (1H, dd, ²J_{gem} = 14.6 Hz, ³J = 8.7 Hz), 3.10 (1H, m), 3.34 - 3.45 (2H, m), 7.16 - 7.21 (4H, m), 7.31 (1H, d, ⁴J = 1.2 Hz), 8.82 (1 H, d, ⁴J = 1.4 Hz)

### EXAMPLE 9

### 4-[(3-Benzyl-2,3-dihydro-1H-inden-1-yl)methyl]-1H-imidazole

### (a) 3-Benzylindan-1-one

This compound was prepared according to the method described by B. M. Trost and L. H. Latimer in J. Org. Chem. 42 (1977) 3212. The starting compounds were 1-indanone and benzyl bromide.

¹H NMR (CDCl₃): δ 2.44 (1H, dd, ²J_{gem} = 19.2 Hz, ³J = 3.1 Hz), 2.76 (1H, dd, ²J_{gem} = 19.2 Hz, ³J = 7.5 Hz), 2.82 (1H, dd, ²J_{gem} = 13.8 Hz, ³J = 9.1 Hz), 3.17 (1H, dd, ²J_{gem} = 13.8 Hz, ³J = 6.0 Hz), 3.72 (1 H, m), 7.16 - 7.19 (2H, m), 7.22 - 7.41 (5H, m), 7.57 (1H, td, Jₒᵣₜₒ = 7.5 Hz, Jₘₑₜₐ = 1.2 Hz), 7.74 (1H, d, Jₒᵣₜₒ = 7.6 Hz)

### (b) 1-Benzyl-5-(3-benzylindan-1-ylidenemethyl)-1H-imidazole

1-Benzyl-5-(3-benzylindan-1-ylidenemethyl)-1H-imidazole was prepared as 1-benzyl-5-(2-phenylindan-1-ylidenemethyl)-1H-imidazole above except that 3-benzylindan-1-one was used as a starting material. The crude product as its hydrochloride salt was used in the next step without purification.

### (c) 4-[(3-Benzyl-2,3-dihydro-1H-inden-1-yl)methyl]-1H-imidazole

4-[(3-Benzyl-2,3-dihydro-1H-inden-1-yl)methyl]-1H-imidazole was prepared as 4-[(2,3-dihydro-6-methoxy-2-phenyl-1H-inden-1-yl)methyl]-1H-imidazol above except that 1-benzyl-5-(3-benzylindan-1-ylidenemethyl)-1H-imidazole was used as a starting material. The crude product which is the mixture of cis and trans diastereomers of 4-[(3-benzyl-2,3-dihydro-1H-inden-1-yl)methyl]-1H-imidazole was purified by flash chromatography (eluent: 9.75:0.25 (v/v) mixture of dichloromethane-methanol). The free base was converted to its hydrochloride salt which was the mixture of the diastereomers (cis:trans 86:14).

The cis diastereomer as base: ¹H NMR (CDCl₃): δ 1.40 (1H, dt, ²J_{gem} = 12.7 Hz, ³J = 9.1 Hz), 2.29 (1H, dt, ²J_{gem} = 12.7 Hz, ³J = 7.5 Hz), 2.58 (1H, dd, ²J_{gem} = 13.7 Hz, ³J = 9.5 Hz), 2.75 (1 H, dd, ²J_{gem} = 14.8 Hz, ³J = 8.5 Hz), 3.19 (1H, dd, ²J_{gem} = 15.4 Hz, ³J = 5.2 Hz), 3.25 (1H, dd, ²J_{gem} = 13.7 Hz, ³J = 5.3 Hz), 3.36 - 3.47 (2H, m), 6.77 (1 H, d, ⁴J = 0.7 Hz), 7.14 - 7.31 (9H, m), 7.53 (1H, d, ⁴J = 0.8 Hz)

### EXAMPLE 10

### 4-(4-Phenyl-1,2,3,4-tetrahydronaphthalen-1-yl)-1H-imidazole

### (a) 1-(3-Benzyl-3H-imidazol-4-yl)-4,4-diphenylbutan-1-ol

Magnesium turnings (0.5 g) were covered with dry tetrahydrofuran (4 ml). 1-Bromo-3,3-diphenylpropane (5.6 g) in 20 ml of dry tetrahydrofuran was added dropwise. The mixture was stirred at reflux for one hour. After being cooled to room temperature, 3-benzyl-3H-imidazole-4-carbaldehyde (3.8 g) in 20 ml of dry tetrahydrofuran was added dropwise to the Grignard reagent and the mixture was refluxed for two hours. The cooled reaction mixture was poured into a cold diluted hydrochloric acid solution. Work-up of the mixture gave the crude product, which was converted to its hydrochloride salt in ethyl acetate using dry hydrochloric acid.

¹H NMR (as HCl-salt, DMSO-d₆): 1.51-1.59 (m, 2H), 1.86-1.92 (m, 1H), 1.99-2.06 (m, 1H), 3.78 (t, J=7.9 Hz, 1H), 4.51 (m, 1H), 5.36 (s, 2H), 5.51 (s, 1H), 7.14-7.39 (m, 16H), 8.46 (s, 1H)

### (b) 1-(1H-Imidazol-4-yl)-4,4-diphenylbutan-1-ol

3.0 g of 1-(3-benzyl-3H-imidazol-4-yl)-4,4-diphenylbutan-1-ol was dissolved in 150 ml of ethanol. The solution was hydrogenated at 45 °C with 10 % palladium on carbon as catalyst for 5 hours. The reaction mixture was filtered, and the filtrate was evaporated to dryness under reduced pressure.

¹H NMR (as HCl-salt, DMSO-d₆): 1.58-1.64 (m, 2H), 1.91-2.01 (m, 1H), 2.08-2.17 (m, 1H), 3.91 (t, J=7.9 Hz, 1H), 4.65 (t, J=6.4 Hz, 1H), 5.49 (s, 1H), 7.12-7.28 (m, 11 H), 8.51 (s, 1H)

### (c) 4-(4-Phenyl-1,2,3,4-tetrahydronaphthalen-1-yl)-1H-imidazole

A mixture of 1-(1H-imidazol-4-yl)-4,4-diphenylbutan-1-ol hydrochloride (2.0 g) and methanesulfonic acid (40 ml) was heated at 100 °C for 40 minutes. The cooled reaction mixture was poured into water and was made alkaline with sodium hydroxide solution. The product was extracted into ethyl acetate, which was washed with water, dried with sodium sulfate and evaporated under reduced pressure to give a crude product of racemic cis and trans diastereomers. The product was purified by flash chromatography (elution with a dichloromethane - methanol gradient). The base product was dissolved in ethyl acetate and converted to its hydrochloride salt with hydrogen chloride gas.

¹H NMR (trans isomer as HCl-salt, MeOH-d₄): 1.93-2.04 (m, 2H), 2.21-2.28 (m, 2H), 4.25-4.29 (m, 1H), 4.52-4.56 (m, 1H), 6.90-7.31 (m, 10H), 8.80 (d, J=1,4 Hz, 1H)

¹H NMR (cis isomer as HCl-salt, MeOH-d₄): 1.74-1.85 (m, 2H), 2.08-2.18 (m, 2H), 4.16-4.20 (m, 1H), 4.42-4.45 (m, 1H), 6.88-7.30 (m, 10H), 8.84 (d, J=1,4 Hz, 1H)

### EXAMPLE 11

### 4-[3-(4-Fluorophenyl)-2,3-dihydro-1H-inden-1-yl]-1H-imidazole

### (a) trans-3-(4-Fluorophenyl)-1-phenylpropenone

A solution of 3.8 g (0.095 mol) of sodium hydroxide in 38 ml of water was dropped gradually into the solution of acetophenone (9.0 g, 0.075 mol) and 4-fluorobenz-aldehyde (9.4 g, 0.076 mol) in ethanol (20 ml). The mixture was stirred for 2 hr at room temperature. Water (80 ml) was added and the mixture was neutralized with 6 M HCl solution. The precipitated trans-3-(4-fluorophenyl)-1-phenylpropenone was filtered, washed with water and dried.

¹H NMR (DMSO-d₆): δ 7.31 (2H, t, ³J = 8.9 Hz), 7.59 (2H, t, ³J = 7.5 Hz), 7.68 (1H, t, ³J = 7.3 Hz), 7.76 (1H, d, ³Jₜᵣₐₙₛ = 15.7 Hz), 7.92 (1H, d, ³Jₜᵣₐₙₛ =15.5 Hz), 7.99 (2H, m), 8.16 (2H, m)

### (b) 3-(4-Fluorophenyl)indan-1-one

Polyphosphoric acid (102 g) was heated in an oil bath at 140 °C and 3-(4-fluorophenyl)-1-phenylpropenone (5.9 g) was added. Heating was continued for 30 min at 140 °C. The mixture was cooled to 80 °C and water was added carefully. The mixture was extracted with ethyl acetate. The organic extracts were washed with water. After drying over sodium sulfate the solvent was evaporated under reduced pressure. The 3-(4-fluorophenyl)indan-1-one obtained was recrystallized from heptane-ethyl acetate 8:2.

¹H NMR (CDCl₃): δ 2.64 (1H, dd, ²J_{gem} = 19.2 Hz, ³J = 3.9 Hz), 3.23 (1H, dd, ²J_{gem} = 19.2 Hz, ³J = 8.1 Hz), 4.57 (1H, dd, ³J = 8.0 Hz, ³J = 3.9 Hz), 7.00 (2H, distorted t, ³J = 8.7 Hz), 7.06 - 7.11 (2H, m), 7.25 (1 H, m), 7.43 (1H, t, Jₒᵣₜₒ = 7.4 Hz), 7.58 (1H, td, Jₒᵣₜₒ = 7.5 Hz, Jₘₑₜₐ = 1.2 Hz), 7.82 (1H, d, Jₒᵣₜₒ = 7.7 Hz)

### (c) 3-(4-Fluorophenyl)-1-(1-trityl-1H-imidazol-4-yl)indan-1-ol

A 3.0 M solution of ethylmagnesium bromide (5.9 ml, 0.0177 mol) in diethyl ether was added to a solution of 4-iodo-1-trityl-1H-imidazole (7.22 g, 0.0165 mol, prepared according to K. L. Kirk J. Heterocycl. Chem. 22 (1985) 57) in 70 ml of dry methylene chloride at ambient temperature. After one hour, a solution of 3-(4-fluorophenyl)indan-1-one (2.00 g, 0.00884 mol) in 6 ml of dry methylene chloride was added and stirring was continued for 45 hr. Saturated ammonium chloride solution was added to quench the reaction. The methylene chloride phase was separated and the aqueous phase was extracted twice with methylene chloride. The combined organic extracts were washed with brine, dried and concentrated. The crude 3-(4-fluorophenyl)-1-(1-trityl-1H-imidazol-4-yl)indan-1-ol was purified by flash chromatography using methylene chloride as an eluent.

### (d) 4-[3-(4-Fluorophenyl)-3H-inden-1-yl]-1H-imidazole

3-(4-Fluorophenyl)-1-(1-trityl-1H-imidazol-4-yl)indan-1-ol (2.22 g) in 22 ml of a 2 M HCl solution was heated at 70 °C for 2 hr. Water was added. The mixture was extracted with methylene chloride. Then methylene chloride phase was extracted with 2 M HCl solution. All combined water layers were made basic and extracted with methylene chloride. The organic phase was washed with water and dried. The solvent was removed under reduced pressure. The crude 4-[3-(4-fluorophenyl)-3H-inden-1-yl]-1H-imidazole which was the mixture of isomers (the ratio 73:27) was purified by flash chromatography (elution with a dichloromethane-methanol gradient).

### (e) 4-[3-(4-Fluorophenyl)-2,3-dihydro-1H-inden-1-yl]-1H-imidazole

The mixture of the isomers of 4-[3-(4-fluorophenyl)-3H-inden-1-yl]-1H-imidazole was hydrogenated in ethanol using 10 % palladium on charcoal as a catalyst. The mixture was filtered through Celite, and the solvent was evaporated. The crude 4-[3-(4-fluorophenyl)-2,3-dihydro-1H-inden-1-yl]-1H-imidazole which was the mixture of the cis and trans diastereomers (the ratio 95.5:4.5) was purified by flash chromatography (elution with a dichloromethane-methanol gradient).

The cis diastereomer as base: ¹H NMR (CDCl₃): δ 2.14 (1H, dt, ²J_{gem} = 11.1 Hz, ³J = 11.0 Hz), 2.89 (1H, dt, ²J_{gem} = 12.3 Hz, ³J = 7.1 Hz), 4.30 (1H, dd, ³J = 10.8 Hz, ³J = 7.2 Hz), 4.44 (1H, dd, ³J = 10.8 Hz, ³J = 7.2 Hz), 6.87 - 6.90 (2H, m), 6.96 (2H, t, ³J = 8.7 Hz), 7.12 - 7.19 (5H, m), 7.43 (1H, s)

### EXAMPLE 12

### 4-(3-Benzyl-2,3-dihydro-1H-inden-1-yl)-1H-imidazole

### 3-Benzyl-1-(1-trityl-1H-imidazol-4-yl)indan-1-ol

This compound was obtained from 4-iodo-1-trityl-1H-imidazole and 3-benzylindan-1-one by the method described for 3-(4-fluorophenyl)-1-(1-trityl-1H-imidazol-4-yl)indan-1-ol as stated above. The 3-benzyl-1-(1-trityl-1H-imidazol-4-yl)indan-1-ol was purified by flash chromatography (the eluent: heptane-ethyl acetate 1:1).

### 4-(3-Benzyl-3H-inden-1-yl)-1H-imidazole

Triethylsilane (1 ml, 0.728 g, 6.26 mmol) and trifluoroacetic acid (1.9 ml, 2.81 g, 24.7 mmol) were added to the solution of 3-benzyl-1-(1-trityl-1H-imidazol-4-yl)indan-1-ol (0.387 g, 0.73 mmol) in dichloromethane (13 ml). The reaction was stirred at room temperature for 20 hr. Then the reaction was quenched with water and made basic with the 2 M sodium hydroxide solution. The dichloromethane layer was washed with water and dried over sodium sulfate. The solvent was removed under reduced pressure. Flash chromatography using a dichloromethane―methanol gradient afforded 4-(3-benzyl-3H-inden-1-yl)-1H-imidazole.

¹H NMR (CDCl₃): δ 2.69 (1 H, dd, ²J_{gem} = 13.5 Hz, ³J = 9.4 Hz), 3.13 (1H, dd, ²J_{gem} = 13.5 Hz, ³J = 6.7 Hz), 3.80 (1H, m), 6.64 (1H, d, ³J = 2.2 Hz), 7.16 - 7.33 (7H, m), 7.37 (1H, s), 7.60 (1H, d, ³J = 0.6 Hz), 7.69 (1H, d, Jₒᵣₜₒ = 7.6 Hz)

### 4-(3-Benzyl-2,3-dihydro-1H-inden-1-yl)-1H-imidazole

4-(3-benzyl-3H-inden-1-yl)-1H-imidazole was hydrogenated in ethanol using 10 % palladium on charcoal as a catalyst. The mixture was filtered through Celite, and the solvent was evaporated. The crude 4-(3-benzyl-2,3-dihydro-1H-inden-1-yl)-1H-imidazole which was the mixture of the cis and trans diastereomers (the ratio 94:6) was purified by flash chromatography (elution with a dichloromethane-methanol gradient).

The cis diastereomer as base: ¹H NMR (CDCl₃): δ 1.82 (1H, dt, ²J_{gem} = 12.5 Hz, ³J = 9.7 Hz), 2.53 (1H, dt, ²J_{gem} = 12.5 Hz, ³J = 7.4 Hz), 2.69 (1H, dd, ²J_{gem} = 13.6 Hz, ³J = 9.4 Hz,), 3.31 (1H, dd, ²J_{gem} = 13.6 Hz, ³J = 5.3 Hz), 3.49 (1H, m), 4.31 (1H, m), 6.80 (1H, s), 7.08 (1H, distorted d, Jₒᵣₜₒ = 7.4 Hz), 7.15 - 7.30 (8H, m), 7.46 (1H, m)

### EXAMPLE 13

### 4-(2,3-Dihydro-3-phenylinden-1-yl)-1H-imidazole

### 2-(tert-Butyldimethylsilanyl)-5-(2,3-dihydro-1-hydroxy-3-phenyl-1H-inden-1-yl)-1H-imidazole-1-sulfonic acid dimethylamide

A solution of imidazole-1-sulfonic acid dimethylamide (1.96 g, 0.0112 mol, prepared according to D. J. Chadwick and R. I. Ngochindo J. Chem. Soc. Perkin Trans. I (1984) 481) in dry tetrahydrofuran (90 ml) under nitrogen was cooled to -78 °C and treated dropwise with 15 % n-butyllithium in hexane (8.2 ml, 0.01393 mol). After 30 minutes tert-butyldimethylsilyl chloride (2.1 g, 0.01393 mol) in dry tetrahydrofuran (5 ml) was added and the mixture was allowed to warm to room temperature. After 1.5 hr the mixture was again cooled to -78 °C and treated with 15 % n-butyllithium in hexane (8.5 ml, 0.01360 mol). After 30 minutes 3-phenyl-1-indanone (3.40 g, 0.01633 mol) in dry tetrahydrofuran was added and the mixture was allowed to warm to room temperature during the night. The mixture was quenched with saturated sodium carbonate solution and the solvent was removed under reduced pressure. The residue was dissolved in dichloromethane and washed twice with water, dried, filtered and concentrated under reduced pressure. Purification was done by flash chromatography (elution with a heptane―ethyl acetate gradient).

¹H NMR (CDCl₃): δ 0.40 (3H, s), 0.41 (3H, s), 0.98 (9H, s), 2.47 (1H, dd, ²J_{gem} = 12.9 Hz, ³J = 10.0 Hz), 2.88 (6H, s), 3.24 (1H, dd, ²J_{gem} = 12.9 Hz, ³J = 7.2 Hz), 4.05 (1H, dd, ³J = 9.9 Hz, ³J = 7.3 Hz), 6.14 (1H, s), 6.92 (1H, d, Jₒᵣₜₒ = 7.0 Hz), 7.20 - 7.36 (7H, m), 7.52 (1H, d, Jₒᵣₜₒ = 7.0 Hz)

### 5-(2,3-Dihydro-1-hydroxy-3-phenyl-1H-inden-1-yl)-1H-imidazole-1-sulfonic acid dimethylamide

A 1.1 M solution of tetrabutylammonium fluoride in tetrahydrofuran (1.4 ml, 1.54 mmol) was added dropwise to the solution of 2-(tert-butyldimethylsilanyl)-5-(2,3-dihydro-1-hydroxy-3-phenyl-1H-inden-1-yl)-1H-imidazole-1-sulfonic acid dimethylamide (637 mg, 1.28 mmol) in tetrahydrofuran (13 ml). The reaction was strirred overnight at room temperature. The reaction was quenched with water and then extracted with ethyl acetate. The organic layer was washed with water and brine. The organic phase was dried and the solvent removed under reduced pressure. The product was recrystallized in ethyl acetate.

¹H NMR (CDCl₃): δ 2.47 (1H, dd, ²J_{gem} = 13.0 Hz, ³J = 10.0 Hz), 3.04 (6H, s), 3.31 (1H, dd, ²J_{gem} = 12.9 Hz, ³J = 7.2 Hz), 4.09 (1H, dd, ³J = 9.9 Hz, ³J = 7.2 Hz), 6.07 (1H, s), 6.96 (1H, d, Jₒᵣₜₒ = 7.6 Hz), 7.19 - 7.27 (4H, m), 7.30 - 7.40 (3H, m), 7.54 (1H, d, Jₒᵣₜₒ = 6.8 Hz), 7.94 (1H,s)

### 5-(3-Phenyl-3H-inden-1-yl)-1H-imidazole-1-sulfonic acid dimethylamide

Triethylsilane (760 µl, 554 mg, 4.77 mmol) and trifluoroacetic acid (1.43 ml, 2.12 g, 18.6 mmol) were added to the solution of 5-(2,3-dihydro-1-hydroxy-3-phenyl-1H-inden-1-yl)-1H-imidazole-1-sulfonic acid dimethylamide (230 mg, 0.60 mmol) in dichloromethane (8 ml). The reaction was stirred overnight at room temperature. Then the reaction was quenched with water and made basic with the 2 M sodium hydroxide solution. The dichloromethane layer was washed with water and dried over sodium sulfate. The solvent was removed under reduced pressure. Purification by flash chromatography using a dichloromethane―methanol gradient afforded the mixture of the isomers (the ratio 65:35) of 5-(3-phenyl-3H-inden-1-yl)-1H-imidazole-1-sulfonic acid dimethylamide.

### 4-(3-Phenyl-3H-inden-1-yl)-1H-imidazole

5-(3-Phenyl-3H-inden-1-yl)-1H-imidazole-1-sulfonic acid dimethylamide (158 mg, 0.43 mmol) in 5 ml of a 1.5 M HCl solution was refluxed for 1.5 hr. The reaction mixture was made basic and then extracted with ethyl acetate. The organic phase was washed with water and dried. The solvent was removed under reduced pressure. The crude 4-(3-phenyl-3H-inden-1-yl)-1H-imidazole which was the mixture of isomers (the ratio 75:25) was purified by flash chromatography (elution with dichloromethane-methanol 9.75:0.25).

### 4-(2,3-Dihydro-3-phenylinden-1-yl)-1H-imidazole

The mixture of the isomers of 4-(3-phenyl-3H-inden-1-yl)-1H-imidazole was hydrogenated in acetic acid at 50 °C using 10 % palladium on charcoal as a catalyst. The mixture was filtered through Celite, and the solvent was evaporated. Water was added and the solution was made basic. The water solution was extracted with ethyl acetate. The organic phase was washed with water, dried and the solvent was evaporated. The hydrochloride salt of the product was made in ethyl acetate. The product 4-(2,3-dihydro-3-phenylinden-1-yl)-1H-imidazole was the mixture of the cis and trans diastereomers (the ratio 95:5).

The cis diastereomer as its hydrochloride salt: ¹H NMR (CD₃OD): δ 2.19 (1H, dt, ²J_{gem} = 12.2 Hz, ³J = 11.0 Hz), 2.99 (1H, dt, ²J_{gem} = 12.2 Hz, ³J = 7.1 Hz), 4.43 (1H, dd, ³J = 10.9 Hz, ³J = 7.0 Hz), 4.66 (1H, dd, ³J = 11.0 Hz, ³J = 7.0 Hz), 6.92 - 6.94 (1H, m), 7.07 - 7.09 (1H, m), 7.23 - 7.37 (7H, m), 7.52 (1H, d, ⁴J = 1.2 Hz), 8.89 (1H, d, ⁴J = 1.4 Hz)

### EXAMPLE 14

### 4-[(1,2,3,4-Tetrahydro-3-phenylnaphthalen-1-yl)methyl]-1H-imidazole

### 1-Benzyl-5-(3-phenyl-3,4-dihydro-2H-naphthalen-1-ylidenemethyl)-1H-imidazole

1-Benzyl-5-(3-phenyl-3,4-dihydro-2H-naphthalen-1-ylidenemethyl)-1H-imidazole was prepared as 1-benzyl-5-(2-phenylindan-1-ylidenemethyl)-1H-imidazole above except that 3-phenyl-3,4-dihydro-2H-naphthalen-1-one (prepared according to J. Vebrel and R. Carrie Bull. Soc. Chem. Fr. (1982) 116) was used as a starting material. The crude product as its hydrochloride salt was used in the next step without purification.

### 4-[(1,2,3,4-Tetrahydro-3-phenylnaphthalen-1-yl)methyl]-1H-imidazole

4-[(1,2,3,4-Tetrahydro-3-phenylnaphthalen-1-yl)methyl]-1H-imidazole was prepared as 4-[(2,3-dihydro-6-methoxy-2-phenyl-1H-inden-1-yl)methyl]-1H-imidazole above except that 1-benzyl-5-(3-phenyl-3,4-dihydro-2H-naphthalen-1-ylidenemethyl)-1H-imidazole was used as a starting material. The hydrochloride salt of the product was the mixture of two diastereomers (82:18, mp. 198 °C).

## Claims

1. An imidazole derivative of formula (I): 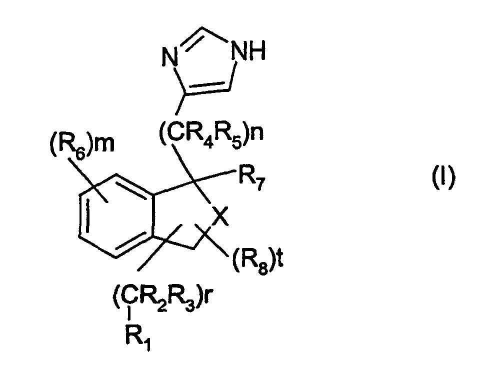 wherein X is -CH₂-(CH₂)ₚ-, -O-, =NH or -S-;
R₁ is phenyl, naphthyl, 1,2,3,4-tetrahydronaphthyl, C₃-C₇-cycloalkyl, C₅ -C₇-cycloalkenyl, C₅ -C₇-cycloalkynyl or mono- or bicyclic aromatic or partially or fully saturated heterocyclic group with 5 to 10 ring atoms which consist of carbon atoms and one to three heteroatoms selected from N, O and S;
wherein the said phenyl, naphthyl,1,2,3,4-tetrahydronaphthyl, C₃ -C₇-cycloalkyl, C₅-C₇-cycloalkenyl, C₅ -C₇-cycloalkynyl or mono- or bicyclic aromatic or partially or fully saturated heterocyclic group is optionally substituted with one to three substituents selected independently from halogen, -OH, -NH₂, halo-C₁-C₆-alkyl, C₁-C₆-alkyl, C₂-C₆-alkenyl, C₂-C₆-alkynyl, C₁-C₆-alkoxy, OH-(C₁-C₆)-alkyl, NH₂-(C₁-C₆)-alkyl and mono- or di(C₁-C₆-alkyl)amino;
R₂ is H or C₁-C₆-alkyl;
R₃ is H or C₁-C₆-alkyl;
R₄ is H or C₁-C₆-alkyl;
R₅ is H, or R₅ and R₇ form together a bond;
each R₆ is independently halogen, -OH, -NH₂, halo-C₁-C₆-alkyl, C₁-C₆-alkyl, C₁-C₆-alkoxy or.OH-( C₁-C₆)-alkyl;
R₇ is H, OH or C₁-C₄-alkyl, or R₇ and R₅ form together a bond;
each R₈ is independently OH, C₁-C₆-alkyl, halo-C₁-C₆-alkyl or C₁-C₆-alkoxy;
m is 0, 1, 2 or 3;
n is 0 or 1;
p is 0 or 1;
r is 0 or 1; and
t is 0, 1 or 2;
or a pharmaceutically acceptable ester or salt thereof.

2. A compound according to claim 1, wherein X is -CH₂-(CH₂)ₚ- and p is 0.

3. A compound according to claim 1, wherein X is -CH₂-(CH₂)ₚ- and p is 1.

4. A compound according to claim 1, wherein X is -O-.

5. A compound according to claim 1, which is a compound of formula 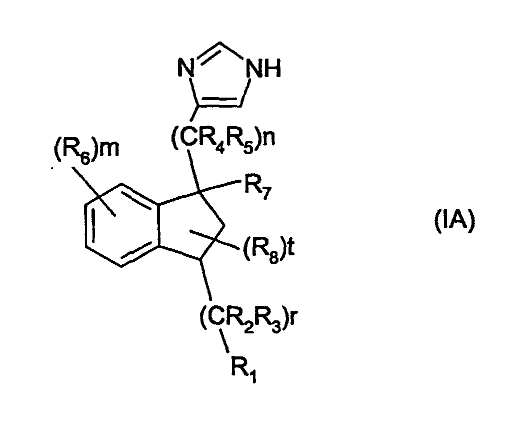 wherein R₁ to R₈, m, n, r and t are as defined in claim 1.

6. A compound according to claim 1, which is a compound of formula 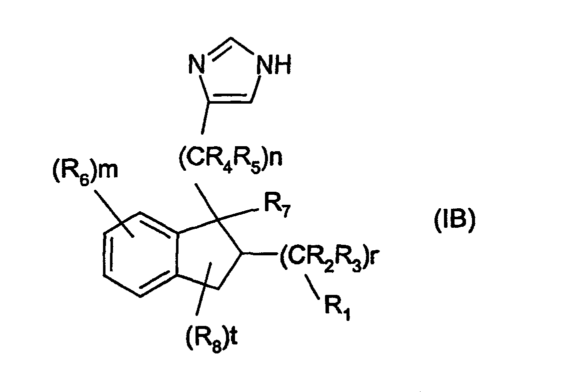 wherein R₁ to R₈, m, n, r and t are as defined in claim 1.

7. A compound according to any of claims 1 to 6, wherein r is 0.

8. A compound according to any one of claims 1 to 6, wherein r is 1 and R₂ and R₃ are H.

9. A compound according to any one of claims 1 to 8, wherein n is 0.

10. A compound according to any one of claims 1 to 8, wherein n is 1.

11. A compound according to any one of claims 1 to 8, wherein n is 1 and R₄ and R₅ are H.

12. A compound according to any one of claims 1 to 11, wherein R₇ is H.

13. A compound according to any one of claims 1 to 12, wherein R₁ is phenyl, naphthyl, pyridyl, thienyl, furyl or cyclohexyl each of which is optionally substituted with one to three of the substituents selected independently from halogen, -OH, -NH₂, halo-C₁-C₆-alkyl, C₁-C₆-alkyl, C₂-C₆-alkenyl, C₂-C₆-alkynyl, C₁-C₆-alkoxy, OH-(C₁-C₆)-alkyl, NH₂-(C₁-C₆)-alkyl and mono- or di(C₁-C₆-alkyl)amino.

14. A compound according to any one of claims 1 to 13, wherein R₁ is phenyl, naphthyl, pyridyl, thienyl, furyl or cyclohexyl each of which is optionally substituted with one to three of the substituents selected independently from halogen, -OH, C₁-C₆-alkoxy and C₁-C₆-alkyl.

15. A compound according to any one of claims 1 to 13, wherein R₁ is phenyl, pyridyl or cyclohexyl each of which is optionally substituted with one to three of the substituents selected independently from halogen, -OH, C₁-C₆-alkoxy and C₁-C₆-alkyl.

16. A compound according to any one of claims 1 to 13, wherein R₁ is phenyl or cyclohexyl which is optionally substituted with one to three of the substituents selected independently from halogen, -OH, -NH₂, halo-C₁-C₆-alkyl, C₁-C₆-alkyl, C₂-C₆-alkenyl, C₂-C₆-alkynyl, C₁-C₆-alkoxy, OH-(C₁-C₆)-alkyl, NH₂-(C₁-C₆)-alkyl and mono- or di(C₁-C₆-alkyl)amino.

17. A compound according to any one of claims 1 to 16, wherein m is 0.

18. A compound according to any one of claims 1 to 17, wherein m is 1 or 2 and each R₆ is selected independently from halogen, -OH, C₁-C₆-alkoxy and C₁-C₆-alkyl.

19. A compound according to any one of claims 1 to 18, wherein t is 0.

20. A pharmaceutical composition comprising a compound according to any one of claims 1 to 19 or a pharmaceutically acceptable ester or salt thereof and optionally a pharmaceutically acceptable carrier, diluent and/or excipient.

21. A compound according to any one of claims 1 to 19 or a pharmaceutically acceptable ester or salt thereof for use in the treatment of neurological, psychiatric or cognition disorders.

22. A compound according to any one of claims 1 to 19 or a pharmaceutically acceptable ester or salt thereof for use in the treatment of diabetes, lipolytic disorders, orthostatic hypotension or sexual dysfunction.

23. Use of a compound according to any one of claims 1 to 19 or a pharmaceutically ester or salt thereof in the manufacture of a medicament for the treatment of neurological, psychiatric or cognition disorders, diabetes, lipolytic disorders, orthostatic hypotension or sexual dysfunction.

24. A process for the preparation of the compounds of formula I according to claim 1 wherein n is 1, **characterized in** reacting a compound of formula II 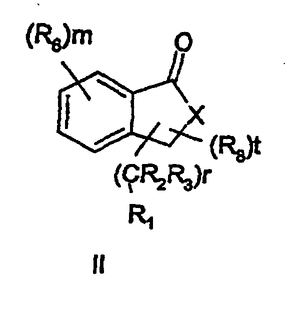 wherein X, R₁ to R₃, R₆, R₈, m, r and t are as define in claim 1, with a compound of formula III 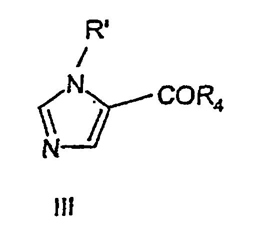 wherein R₄ is as defined in claim 1 and R' is H or a protecting group, to obtain a compound of formula I', 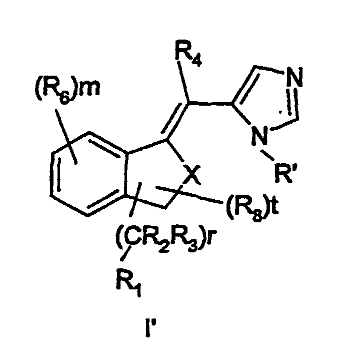 wherein X, R₁ to R₄, R₆, R₈, m, r, t and R' are as defined above,
which is then deprotected and isolated in a conventional manner, or which is converted by hydrogenation to another compound of formula I, 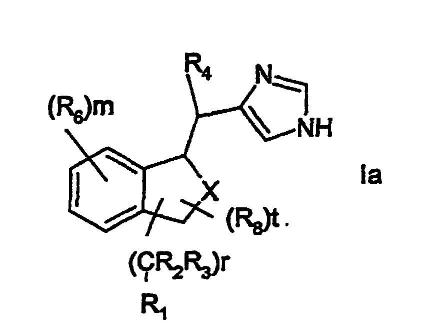 wherein X, R₁ to R₄, R₆, R₈, m, r, t and R' are as defined above.

## Patentansprüche

1. Imidazolderivat der Formel (I): 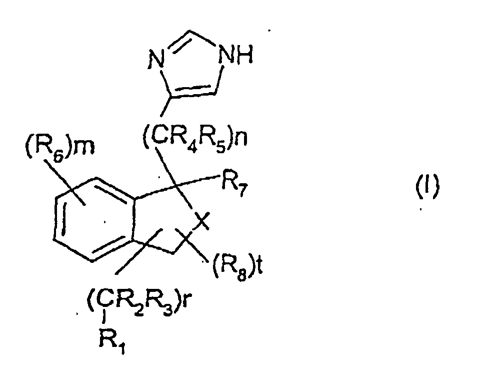 worin X -CH₂-(CH₂)ₚ-, -O-, =NH oder -S- ist;
R₁ eine Phenyl-, Naphthyl-, 1,2,3,4-Tetrahydronaphthyl-, C₃-C₇-Cycloalkyl-, C₅-C₇-Cycloalkenyl-, C₅-C₇-Cycloalkinyl- oder eine mono- oder bicyclische aromatische oder teilweise oder vollständig gesättigte heterocyclische Gruppe mit 5 bis 10 Ringatomen ist, die aus Kohlenstoffatomen und ein bis drei Heteroatomen besteht, die ausgewählt sind aus N, O und S;
wobei diese Phenyl-, Naphthyl-, 1,2,3,4-Tetrahydronaphthyl-, C₃-C₇-Cycloalkyl-, C₅-C₇-Cycloalkenyl-, C₅-C₇-Cycloalkinyl- oder diese mono- oder bicyclische aromatische oder teilweise oder vollständig gesättigte heterocyclische Gruppe gegebenenfalls mit ein bis drei Substituenten substituiert ist, die unabhängig ausgewählt sind aus Halogen, -OH, -NH₂, Halo-C₁-C₆-alkyl, C₁-C₆-Alkyl, C₂-C₆-Alkenyl, C₂-C₆-Alkinyl, C₁-C₆-Alkoxy, OH-(C₁-C₆)-alkyl, NH₂-(C₁-C₆)-alkyl und Mono- oder Di(C₁-C₆-alkyl)amino;
R₂ H oder C₁-C₆-Alkyl ist;
R₃ H oder C₁-C₆-Alkyl ist;
R₄ H oder C₁-C₆-Alkyl ist;
R₅ H ist oder R₅ und R₇ zusammen eine Bindung bilden;
jedes R₆ unabhängig Halogen, -OH, -NH₂, Halo-C₁-C₆-alkyl, C₁-C₆-Alkyl, C₁-C₆-Alkoxy oder OH-(C₁-C₆)-alkyl ist;
R₇ H, OH oder C₁-C₄-Alkyl ist oder R₇ und R₅ zusammen eine Bindung bilden;
jedes R₈ unabhängig OH, C₁-C₆-Alkyl, Halo-C₁-C₆-alkyl oder C₁-C₆-Alkoxy ist;
m 0, 1, 2 oder 3 ist;
n 0 oder 1 ist;
p 0 oder 1 ist;
r 0 oder 1 ist; und
t 0, 1 oder 2 ist;
oder ein pharmazeutisch verträglicher Ester oder ein pharmazeutisch verträgliches Salz davon.

2. Verbindung nach Anspruch 1, worin X -CH₂-(CH₂)ₚ- ist und p 0 ist.

3. Verbindung nach Anspruch 1, worin X -CH₂-(CH₂)ₚ- ist und p 1 ist.

4. Verbindung nach Anspruch 1, worin X -O- ist.

5. Verbindung nach Anspruch 1, welche eine Verbindung der Formel 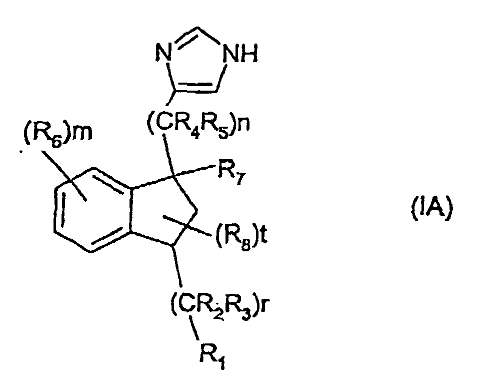 ist, worin R₁ bis R₈, m, n, r und t wie in Anspruch 1 definiert sind.

6. Verbindung nach Anspruch 1, welche eine Verbindung der Formel 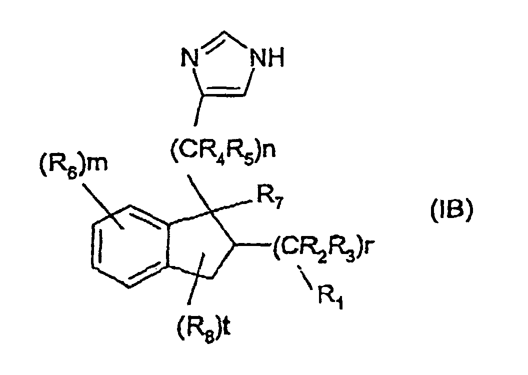 ist, worin R₁ bis R₈, m, n, r und t wie.in Anspruch 1 definiert sind.

7. Verbindung nach irgendeinem der Ansprüche 1 bis 6, worin r 0 ist.

8. Verbindung nach irgendeinem der Ansprüche 1 bis 6, worin r 1 ist und R₂ und R₃ H sind.

9. Verbindung nach irgendeinem der Ansprüche 1 bis 8, worin n 0 ist.

10. Verbindung nach irgendeinem der Ansprüche 1 bis 8, worin n 1 ist.

11. Verbindung nach irgendeinem der Ansprüche 1 bis 8, worin n 1 ist und R₄ und R₅ H sind.

12. Verbindung nach irgendeinem der Ansprüche 1 bis 11, worin R₇ H ist.

13. Verbindung nach irgendeinem der Ansprüche 1 bis 12, worin R₁ Phenyl, Naphthyl, Pyridyl, Thienyl, Furyl oder Cyclohexyl ist, von denen jedes gegebenenfalls mit ein bis drei Substituenten substituiert ist, die unabhängig ausgewählt sind aus Halogen, -OH, -NH₂, Halo-C₁-C₆-alkyl, C₁-C₆-Alkyl, C₂-C₆-Alkenyl, C₂-C₆-Alkinyl, C₁-C₆-Alkoxy, OH-(C₁-C₆)-alkyl, NH₂-(C₁-C₆)-alkyl und Mono- oder Di(C₁-C₆-alkyl)amino.

14. Verbindung nach irgendeinem der Ansprüche 1 bis 13, worin R₁ Phenyl, Naphthyl, Pyridyl, Thienyl, Furyl oder Cyclohexyl ist, von denen jedes gegebenenfalls mit ein bis drei Substituenten substituiert ist, die unabhängig ausgewählt sind aus Halogen, -OH, C₁-C₆-Alkoxy und C₁-C₆-Alkyl.

15. Verbindung nach irgendeinem der Ansprüche 1 bis 13, worin R₁ Phenyl, Pyridyl oder Cyclohexyl ist, von denen jedes gegebenenfalls mit ein bis drei Substituenten substituiert ist, die unabhängig ausgewählt sind aus Halogen, -OH, C₁-C₆-Alkoxy und C₁-C₆-Alkyl.

16. Verbindung nach irgendeinem der Ansprüche 1 bis 13, worin R₁ Phenyl oder Cyclohexyl ist, welches gegebenenfalls mit ein bis drei Substituenten substituiert ist, die unabhängig ausgewählt sind aus Halogen, -OH, -NH₂, Halo-C₁-C₆-alkyl, C₁-C₆-Alkyl, C₂-C₆-Alkenyl, C₂-C₆-Alkinyl, C₁-C₆-Alkoxy, OH-(C₁-C₆)-alkyl, NH₂-(C₁-C₆)-alkyl und Mono- oder Di(C₁-C₆-alkyl)amino.

17. Verbindung nach irgendeinem der Ansprüche 1 bis 16, worin m 0 ist.

18. Verbindung nach irgendeinem der Ansprüche 1 bis 17, worin m 1 oder 2 ist und jedes R₆ unabhängig ausgewählt ist aus Halogen, -OH, C₁-C₆-Alkoxy und C₁-C₆-Alkyl.

19. Verbindung nach irgendeinem der Ansprüche 1 bis 18, worin t 0 ist.

20. Pharmazeutische Zusammensetzung, umfassend eine Verbindung nach irgendeinem der Ansprüche 1 bis 19 oder einen pharmazeutisch verträglichen Ester oder ein pharmazeutisch verträgliches Salz davon und gegebenenfalls einen pharmazeutisch verträglichen Träger, ein pharmazeutisch verträgliches Verdünnungsmittel und/oder einen pharmazeutisch verträglichen Hilfsstoff.

21. Verbindung nach irgendeinem der Ansprüche 1 bis 19 oder ein pharmazeutisch verträglicher Ester oder ein pharmazeutisch verträgliches Salz davon zur Verwendung bei der Behandlung von neurologischen Störungen, psychiatrischen Störungen oder Kognitionsstörungen.

22. Verbindung nach irgendeinem der Ansprüche 1 bis 19 oder ein pharmazeutisch verträglicher Ester oder ein pharmazeutisch verträgliches Salz davon zur Verwendung bei der Behandlung von Diabetes, lipolytischen Störungen, orthostatischer Hypotonie oder sexueller Dysfunktion.

23. Verwendung einer Verbindung nach irgendeinem der Ansprüche 1 bis 19 oder eines pharmazeutisch verträglichen Esters oder eines pharmazeutisch verträglichen Salzes davon zur Herstellung eines Medikaments zur Behandlung von neurologischen Störungen, psychiatrischen Störungen oder Kognitionsstörungen, Diabetes, lipolytischen Störungen, orthostatischer Hypotonie oder sexueller Dysfunktion.

24. Verfahren zur Herstellung der Verbindungen der Formel I nach Anspruch 1, worin n 1 ist, **gekennzeichnet durch** die Umsetzung einer Verbindung der Formel II 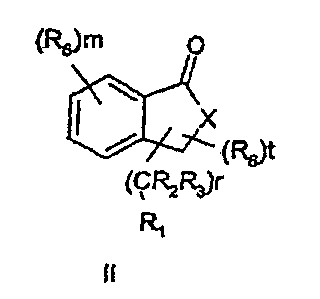 worin X, R₁ bis R₃, R₆, R₈, m, r und t wie in Anspruch 1 definiert sind, mit einer Verbindung der Formel III 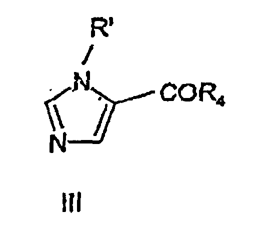 worin R₄ wie in Anspruch 1 definiert ist und R¹ H oder eine Schutzgruppe ist, zu einer Verbindung der Formel I' 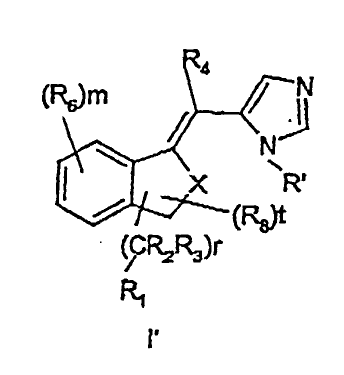 worin X, R₁ bis R₄, R₆, R₈, m, r, t und R' wie oben definiert sind,
welche dann entschützt und auf übliche Weise isoliert wird oder welche **durch** Hydrierung in eine weitere Verbindung der Formel I überführt wird 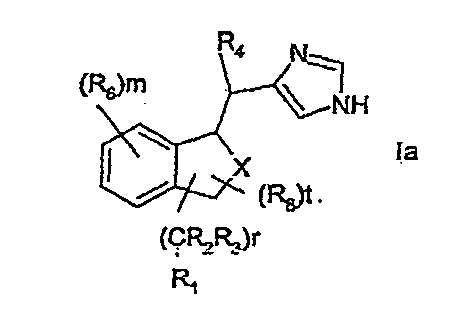 worin X, R₁ bis R₄, R₆, R₈, m, r, t und R' wie oben definiert sind.

## Revendications

1. Dérivé d'imidazole de formule (I) : 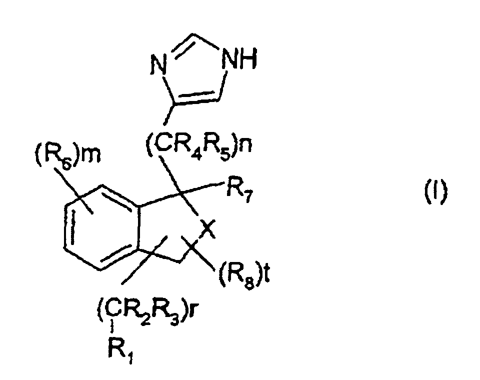 dans laquelle
- X représente un groupe -CH₂-(CH₂)p-, -O-, =NH ou -S- ;
- R₁ représente un groupe phényle, naphtyle, 1,2,3,4-tétrahydronaphtyle, cycloalkyle C₃-C₇, cycloalkényle C₅-C₇, cycloalkynyle C₅-C₇ ou un groupe aromatique mono- ou bi-cyclique ou un groupe hétérocyclique partiellement ou complètement saturé à 5 à 10 atomes de cycle, qui consistent en des atomes de carbone et en un à trois hétéro-atomes choisis parmi N, O et S ;
dans laquelle ledit groupe phényle, naphtyle, 1,2,3,4-tétrahydronaphtyle, cycloalkyle C₃-C₇, cycloalkényle C₅-C₇, cycloalkynyle C₅-C₇ ou aromatique mono- ou bi-cyclique ou hétérocyclique partiellement ou complètement saturé, est éventuellement substitué par un à trois substituants choisis indépendamment parmi les halogènes, les groupes -OH, -NH₂, haloalkyle C₁-C₆, alkyle C₁-C₆, alkényle C₂-C₆, alkynyle C₂-C₆, alcoxy C₁-C₆, OH-alkyle C₁-C₆, NH₂-alkyle C₁-C₆, et mono- ou di-alkyl(C₁-C₆)amino ;
- R₂ représente H ou un groupe alkyle C₁-C₆ ;
- R₃ représente H ou un groupe alkyle C₁-C₆;
- R₄ représente H ou un groupe alkyle C₁-C₆;
- R₅ représente H ou bien R₅ et R₇ en association forment une liaison;
- chaque R₆ représente indépendamment un halogène, un groupe -OH, -NH₂, haloalkyle C₁-C₆, alkyle C₁-C₆, alcoxy C₁-C₆ ou OH-alkyle C₁-C₆;
- R₇ représente H, un groupe OH ou alkyle C₁-C₄, ou bien R₇ et R₅ forment ensemble une liaison ;
- chaque R₈ représente indépendamment un groupe OH, alkyle C₁-C₆, haloalkyle C₁-C₆ ou alcoxy C₁-C₆;
- m est égal à 0, 1, 2 ou 3 ;
- n est égal à 0 ou 1 ;
- p est égal à 0 ou 1 ;
- r est égal à 0 ou 1 ; et
- t est égal à 0, 1 ou 2 ;
ou un ester ou sel pharmaceutiquement acceptable de celui-ci.

2. Composé selon la revendication 1, dans lequel X représente un groupe -CH₂-(CH₂)ₚ- et p est égal à 0.

3. Composé selon la revendication 1, dans lequel X représente un groupe -CH₂-(CH₂)ₚ- et p est égal à 1.

4. Composé selon la revendication 1, dans lequel X représente -O-.

5. Composé selon la revendication 1, qui est un composé de formule : 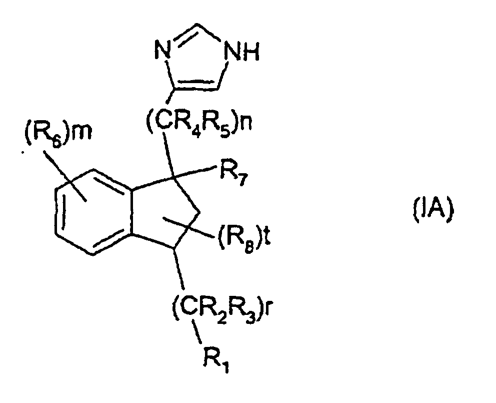 dans laquelle R₁ à R₈, m, n, r et t sont tels que définis dans la revendication 1.

6. Composé selon la revendication 1, qui est un composé de formule : 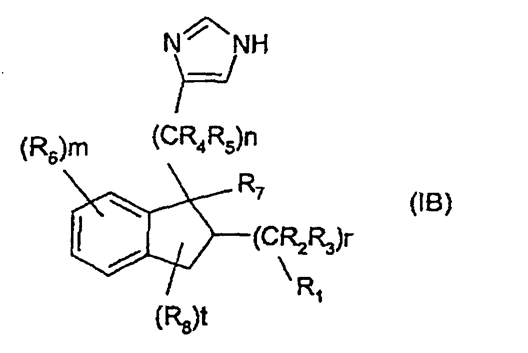 dans laquelle R₁ à R₈, m, n, r et t sont tels que définis dans la revendication 1.

7. Composé selon l'une quelconque des revendications 1 à 6, dans lequel r est égal à 0.

8. Composé selon l'une quelconque des revendications 1 à 6, dans lequel r est égal à 1 et R₂ et R₃ représentent H.

9. Composé selon l'une quelconque des revendications 1 à 8, dans lequel n est égal à 0.

10. Composé selon l'une quelconque des revendications 1 à 8, dans lequel n est égal à 1.

11. Composé selon l'une quelconque des revendications 1 à 8, dans lequel n est égal à 1 et R₄ et R₅ représentent H.

12. Composé selon l'une quelconque des revendications 1 à 11, dans lequel R₇ représente H.

13. Composé selon l'une quelconque des revendications 1 à 12, dans lequel R₁ représente un groupe phényle, naphtyle, pyridyle, thiényle, furyle ou cyclohexyle, chacun d'eux étant éventuellement substitué par un à trois des substituants choisis indépendamment parmi les halogènes, les groupes -OH, -NH₂, haloalkyle C₁-C₆, alkyle C₁-C₆, alkényle C₂-C₆, alkynyle C₂-C₆, alcoxy C₁-C₆, OH-alkyle C₁-C₆, NH₂-alkyle C₁-C₆, et mono- ou di-alkyl(C₁-C₆)amino.

14. Composé selon l'une quelconque des revendications 1 à 13, dans lequel R₁ représente un groupe phényle, naphtyle, pyridyle, thiényle, furyle ou cyclohexyle, chacun d'eux étant éventuellement substitué par un à trois des substituants choisis indépendamment parmi les halogènes, les groupes -OH, alcoxy C₁-C₆ et alkyle C₁-C₆.

15. Composé selon l'une quelconque des revendications 1 à 13, dans lequel R₁ représente un groupe phényle, pyridyle ou cyclohexyle, chacun d'eux étant éventuellement substitué par un à trois des substituants choisis indépendamment parmi les halogènes, les groupes - OH, alcoxy C₁-C₆ et alkyle C₁-C₆.

16. Composé selon l'une quelconque des revendications 1 à 13, dans lequel R₁ représente un groupe phényle ou cyclohexyle, qui est éventuellement substitué par un à trois des substituants choisis indépendamment parmi les halogènes, les groupes -OH, -NH₂, haloalkyle C₁-C₆, alkyle C₁-C₆, alkényle C₂-C₆, alkynyle C₂-C₆, alcoxy C₁-C₆, OH-alkyle C₁-C₆, NH₂-alkyle C₁-C₆, et mono- ou di-alkyl(C₁-C₆)amino.

17. Composé selon l'une quelconque des revendications 1 à 16, dans lequel m est égal à 0.

18. Composé selon l'une quelconque des revendications 1 à 17, dans lequel m est égal à 1 ou à 2 et chaque R₆ est choisi indépendamment parmi les halogènes, les groupes -OH, alcoxy C₁-C₆ et alkyle C₁-C₆.

19. Composé selon l'une quelconque des revendications 1 à 18, dans lequel t est égal à 0.

20. Composition pharmaceutique comprenant un composé selon l'une quelconque des revendications 1 à 19 ou un ester ou sel pharmaceutiquement acceptable de celui-ci, et éventuellement un véhicule, diluant et/ou excipient pharmaceutiquement acceptable.

21. Composé selon l'une quelconque des revendications 1 à 19, ou ester ou sel pharmaceutiquement acceptable de celui-ci, destiné à l'emploi dans le traitement de troubles neurologiques, psychiatriques ou de la connaissance.

22. Composé selon l'une quelconque des revendications 1 à 19, ou ester ou sel pharmaceutiquement acceptable de celui-ci, destiné à l'emploi dans le traitement du diabète, de troubles lipolytiques, de l'hypotension orthostatique ou du dysfonctionnement sexuel.

23. Utilisation d'un composé selon l'une quelconque des revendications 1 à 19, ou d'un ester ou sel pharmaceutiquement acceptable de celui-ci, dans la fabrication d'un médicament pour le traitement de troubles neurologiques, psychiatriques ou de la connaissance, du diabète, de troubles lipolytiques, de l'hypotension orthostatique ou du dysfonctionnement sexuel.

24. Procédé pour la préparation de composés de formule (I) selon la revendication 1 dans laquelle n est égal à 1, caractérisé en la mise en réaction d'un composé de formule (II) 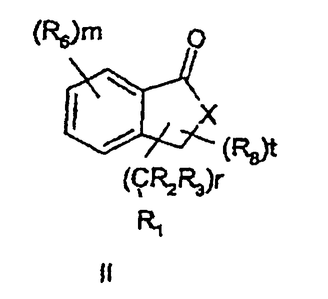 dans laquelle X, R₁ à R₃, R₆, R₈, m, r et t sont tels que définis dans la revendication 1,
avec un composé de formule (III) 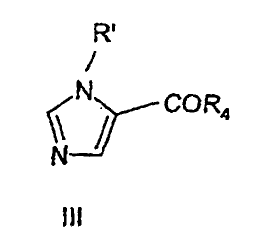 dans laquelle R₄ est tel que défini dans la revendication 1, et R' représente H ou un groupe protecteur, pour obtenir un composé de formule (I'), 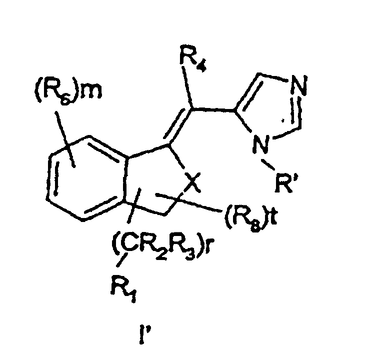 dans laquelle X, R₁ à R₄, R₆, R₈, m, r, t et R' sont tels que définis ci-dessus,
qui est ensuite déprotégé et isolé d'une manière conventionnelle ou qui est converti par hydrogénation en un autre composé de formule (I) 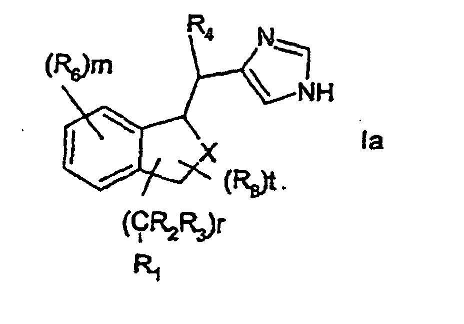 dans laquelle X, R₁ à R₄, R₆, R₈, m, r, t et R' sont tels que définis ci-dessus.
